# EUROPEAN PATENT SPECIFICATION

(11) **EP 2 023 891 B1**
(45) Date of publication and mention of the grant of the patent: **24.01.2018**
(21) Application number: 07794820.6
(22) Date of filing: 11.05.2007
(51) Int. Cl.: A61Q 19/00, A61Q 19/10, A61Q 1/06, A61Q 5/02, A61Q 5/06, A61Q 15/00, A61Q 17/04, A61K 8/81, A61Q 3/02, A61K 8/06, A61Q 1/00, A61Q 1/08

(54) **PERSONAL CARE COMPOSITIONS CONTAINING FUNCTIONALIZED POLYMERS**
KÖRPERPFLEGEZUSAMMENSETZUNGEN MIT FUNKTIONALISIERTEN POLYMEREN
COMPOSITIONS DE SOINS PERSONNELS CONTENANT DES POLYMERES FONCTIONNALISES

(30) Priority: 11.05.2006 US 799616 P; 12.02.2007 US 900847 P; 11.05.2007 US 747261
(43) Date of publication of application: 18.02.2009
(60) Divisional application: 10189923.5
(73) Proprietor: Evonik Degussa GmbH, 45128 Essen (DE)
(72) Inventor: NOOR, Mussarat, Roselle Park, New Jersey 07204 (US); LEMMA, Solomon, Orefield, Pennsylvania 18069 (US)
(86) International application number: PCT/US2007/011486
(87) International publication number: WO 2007/133720

(56) References cited:
- EP-A- 1 466 579
- WO-A-01/19333
- US-A1- 2003 147 946
- US-A1- 2005 031 565
- US-A1- 2005 123 493
- US-A1- 2005 131 565
- US-A1- 2005 137 117
- US-A1- 2005 169 865
- US-A1- 2005 175 570
- US-A1- 2005 261 159

## Description

### BACKGROUND OF THE INVENTION

### FIELD OF THE INVENTION

This invention relates to personal care or cosmetic compositions comprising functionalized polymers.

### INTRODUCTION TO THE INVENTION

U.S. Patent Nos. 4,057,622, 4,057,623, 4,057,624, 5,318,995, 5,519,063 and 5,736,125 disclose the possibility of thickening oil-containing compositions with certain polymers containing (a) lipophilic groups (e.g. in units derived from long chain n-alkyl acrylates) and (b) certain other groups, namely amide groups (in units derived from arylamide), pyrrolidone groups (in units derived from N-vinyl pyrrolidone), imidazole groups (in units derived from N-vinyl imidazole), carboxylic acid and carboxylic acid salt groups(e.g. in units derived from acrylic or Methacrylic acid), sulphonic acid groups, and sulphonic acid salt groups.

Side Chain Crystalline Polymers (SCC) are disclosed in Application Serial Nos. 11/199,049; and 11/199,508. Publications describing SCC polymers include U.S. Patent Nos. 4,830,855, 5,120,349, 5,156,911, 5,387,450, 5,412,035, 5,665,822, 5,783,302, 5,752,926, 5,807,291, 5,469,867, 5,826,584; 6,989,417 (Steven P. Bitler) and 7,101,928 (Steven P. Bitler).

### BRIEF SUMMARY OF THE INVENTION

The present invention relates to a personal care composition as defined in claim 1 and a method to prepare it as set out in claim 16.

A broad range of functionalized side chain crystalline (SCC) polymers can be used to thicken oils, provided that the SCC polymer will dissolve in the oil at a temperature above the crystalline melting point of the polymer (referred to herein as Tₚ) and can crystallize when the solution of the polymer in the oil is cooled to a temperature which is below Tₚ and at which the thickened oil composition is to be used. Without wishing to be bound by any theory or explanation, it is believed that the SCC polymer crystallizes into a network in which the polymer crystallites are connected to one another by semi-soluble chains.

The invention provides a personal care composition and method for making the composition wherein the SCC polymer comprises poly C₈-C₂₂ alkylacrylates/methacrylic acid crosspolymers. Such functionalized SCC polymers can be employed in a wide range of personal care compositions including skin care, body wash, shampoo, hair styling and hair treatments (e.g., hair molding cream, combing cream and pomade, hair sprays, hair colorant), sunscreen, lipstick, anti-perspirant , deodorant, shaving and after shaving products and among other personal care products. The amount of SCC polymer is sufficient to thicken, modify rheology, film form, enhance aesthetics, or improve sensory and feel, especially silicone, among other benefits.

A method of making a composition comprises
(i) dissolving the SCC polymer in the oil at a temperature above Tₚ, and
(ii) cooling the solution to crystallize the polymer in the oil.

An advantage of using these SCC polymers as thickening agents, particularly in water-in-oil emulsions, water-in-silicone, silicone-in-water or multi-phase emulsions, for example, water/oil/water or oil/water/oil, are that the need to use a surface active agent and any other water or oil phase thickener or rheology modifier other than SCC is reduced or removed. Thus the compositions can contain less than 5%, typically less than 2%, usually less than 1%, and in some cases about 0%, of surface active agents or other oil phase or water phase thickeners or rheology modifiers, the percentages being by weight based on the weight of the oil. This is particularly useful in cosmetic and personal care products, since it is conventional for such products to contain surface active agents (for example, perfluoroalkyl organic compounds), and surface active agents can cause an adverse reaction when they contact human skin.

### DETAILED DESCRIPTION OF THE INVENTION

The invention is particularly useful for personal care compositions, for example cosmetics, toiletries, and cleansers, including but not limited to lipsticks, deodorant sticks, nail varnishes, creams, gels and oils, including sunscreen, hand protective products, night renewal products, body milks, creams and lotions, light facial products, protective day care products, liquid moisturizing emulsions, oil-in-water and water-in-oil creams, as well as thickened oil products with or without water and products designed to assist in removing other cosmetic, makeup or personal care products. The invention is also useful in other contexts, for example in paints, film-forming compositions, inks, and compositions carrying active ingredients such as UV absorbers, fragrances, antimicrobial agents, germicides, antioxidants, preservatives, enzymes, nutrients, minerals and if desired, the foregoing can be supplied in a controlled-release format.

The instant invention employs at least one of functionalized side chain crystalline polymers (FSCC or functionalised SCC) to obtain improved cosmetic or personal care formulations, wherein the FSCC polymer comprises at least Poly C8-22 AlkylAcrylates/Methacrylic Acid Crosspolymer (Intelimer® 8600, Intelimer® 8100). The following are examples of some of the benefits that can be obtained by using the FSCC or FSCC and SCC polymers:
1. Ability to modify the rheology of cosmetic emulsion products or anhydrous products by thickening the oil phase,
2. Ability to modify the rheology of cosmetic emulsion products or anhydrous products by thickening the oil phase of silicone oil based formulations,
3. Compatibility in wide variety of cosmetic ingredients,
4. Usage in 100% solid form, or emulsified,
5. Improved water resistance and rub off resistance in cosmetic formulation for skin and hair,
6. Incorporation of active ingredients in the amorphous crystalline matrix structure and ability to release active ingredient in more controlled manner
7. Ability to enhance SPF of sunscreen formulations,
8. Ability to provide breathable film in cosmetic formulations on skin,
9. Film properties can be strengthened and converted into more occlusive (moisture retaining) film in combination of other film forming polymers soluble in oil or water phase along with these polymer
10. Ability to provide fragrance retention and volatile ingredient retention,
11. Ability to make lamellar gels more water resistant without increasing their viscosity,
12. Ability to formulate water-resistant emulsions based on alcohol and/or nonionic ethoxylated emulsifiers,
13. Sensory and texture benefits can be modified and enhanced by using different SCC polymer functionalities, among other benefits that would be understood by a person having ordinary skill in this art.

### Definitions and Abbreviations

In this specification, parts and percentages are by weight, except where otherwise noted. Temperatures are in °C. The onset-of-melting temperature, Tₒ ,the peak melting temperature, Tₚ, and the heat of fusion, J/g, are determined using a differential scanning calorimeter (DSC) at a rate of temperature change of 10 °C./min, for example from -10 to 150 °C., and on the second heat cycle. Tₚ is the temperature at the peak of the DSC curve, and Tₒ is the temperature at the intersection of the baseline of the DSC peak and the onset line, the onset line being defined as the tangent to the steepest part of the DSC curve below Tₚ. The abbreviations Mn and Mw are used to denote number average and weight average molecular weight in daltons, respectively, measured in tetrahydrofuran using size exclusion chromatography, configured with a Wyatt laser light scattering detector. Bulk viscosities given in the Examples for the polymeric thickeners are in centipoise and were measured using a Brookfield LVT viscometer with an electrically thermostatted Thermosel heater, controlled for example to 95 °C., and small sample adapter using spindles 4 and 7. Wt% or %w/w refers to weight percent; and q.s. means to incorporate an amount of the indicated material sufficient to make the sum of all %w/w equal to 100

The abbreviation CxA is used to denote an n-alkyl acrylate in which the n-alkyl group contains x carbon atoms, the abbreviation Cx alkyl is used to denote an n-alkyl group which contains x carbon atoms, and the abbreviation CxM is used to denote an n-alkyl methacrylate in which the n-alkyl group contains x carbon atoms. Other abbreviations are given elsewhere in the specification.

### The Polymeric Thickeners

The FSCC and/or SCC polymers used as thickeners can be homopolymers, or copolymers of two or more comonomers, including random copolymers, graft copolymers and block copolymers (including thermoplastic elastomers). Two or more SCC polymers can be used together. The number average molecular weight of the SCC polymer is generally from about 10,000 to about 1,500,000, normally 12,000 to 1,000,000. The molecular weight of an SCC polymer is relatively unimportant to its Tₚ, but is generally an important factor in determining the Tₚ of other polymers.

The SCC polymer usually melts over a relatively small temperature range. The closer Tₚ is to room temperature, in general the more rapid the transition. The SCC polymer normally has an onset of melting temperature, Tₒ, such that Tₚ - Tₒ is less than Tₚ^{0.7}, generally less than Tₚ^{0.6}, particularly less than 10 °C., especially less than 6 °C., Tₒ and Tₚ being in °C. The crystallinity of the SCC polymer is typically such that its heat of fusion is at least 20 J/g, particularly at least 40 J/g.

The SCC polymer may for example be derived from one or more acrylic, methacrylic, olefinic, epoxy, vinyl, ester-containing, amide-containing or ether-containing monomers. SCC polymers can comprise repeating units in which the side chains comprise linear polymethylene radicals containing 10 to 50, e.g. 16-50, especially 16 to 22, carbon atoms. Polymers containing such units can be prepared by polymerizing a monomer component comprising one or more corresponding linear aliphatic acrylates or methacrylates, or equivalent monomers such as acrylamides or methacrylamides. A number of such monomers are available commercially, either as individual monomers or as mixtures of identified monomers, for example C12A, C14A, C16A, C18A, C22A, a mixture of C18A, C20A and C22A, and a mixture of C26A to C40A. The polymers may also contain units derived from one or more other comonomers, for example straight or branched chain alkyl acrylates or methacrylates in which the alkyl group contains less than 12 carbon atoms, and monomers containing suitable functional groups, for example functional groups comprising at least one member selected from the group consisting of oxygen-, nitrogen- or silicon-containing, carboxy- or hydroxyl- groups, cationic functionality (quaternized compounds such as those containing nitrogen) dimethyl amines (e.g. arylamide), among other functionality. Such monomers include, for example, those listed below. In the list below, the term (meth)acrylate means that the compound may be either an acrylate or a methacrylate:
(a) Nitrogen-containing monomers, for example N,N-dialkylamino (in particular, dimethylamino) (meth)acrylates; ammonium salt-containing (meth)acrylates, for example 2-trimethylammonium methylmethacrylate chloride, methacrylamidopropyl trimethylammonium chloride, N,N- (diethyl or dimethyl)aminoethyl(meth)acrylate methosulfate; imides like the ring-closed reaction products of maleic or itaconic anhydride with primary amines; 2-methacryloxy-N-ethylmorpholine; 2-t-butylaminoethyl methacrylate; (meth)acrylonitrile; t-butylaminoethyl (meth)acrylate; acryloylmorpholine; N-(2-hydroxyethyl)acetamide; 1-piperidinoethyl (meth)acrylate; vinyl pyrollidone; and vinyl pyridines.
(b) Oxygen-containing monomers which are substantially free of carboxylic acid groups, carboxylic acid salt groups, sulphonic acid groups, sulphonic acid salt groups, and amido groups, for example hydroxyalkyl (in particular, hydroxyethyl, hydroxypropyl, and hydroxybutyl) (meth)acrylates; tetrahydrofurfuryl (meth)acrylate; glycidyl methacrylate; alkoxyalkyl (meth)acrylate, e.g. methoxyethyl (meth)acrylate; 1-acryloxy-2-hydroxy-3-phenoxypropane; methylol methacrylate; ethoxyethyl (meth)acrylate; 2-(2-ethoxyethoxy)ethylacrylate; acetoacetoxyethyl (meth)acrylate; phenoxyethyl (meth)acrylate and (meth)acrolein; vinyl alcohol; vinyl ethers; and vinyl esters.
(c) Silicon-containing, e.g. silyl, monomers, for example trimethylsiloxy ethyl(meth)acrylate, 3-acryloxypropyl trimethoxysilane, polydimethyl-siloxane monomethyl methacrylate terminated, and 3-acryloxypropyl tris(trimethylsiloxy)silane.

When the SCC polymer comprises a graft or block copolymer, it can be prepared by copolymerizing a vinyl type macromonomer with other monomers, or by making an SCC polymer, and then reacting the functionalized polymer with the second block material, for example a urethane block, an epoxy block, a polyether block, e.g. a polyethyleneoxide, polypropyleneoxide or polytetramethyleneoxide block, a polysiloxane block, or a poly(alkyl or alkoxy)silane block, or it can be prepared by using any other suitable method of polymerization such as emulsion or suspension polymerization in the presence of surface active agents and/or colloids, or bulk polymerization or solution polymerization. Non-limiting examples of surface active agents comprise at least one member of cetearth, laureth, pareth, PEG and/or PPG or POE esters and ethers of sorbitan Oleate, octyl phenyl and/or ester of nonylphenyl and sulfosuccinate, sodium or Disodium salts like Disodium C-isodecylslfosuccinate, sodium isostearate, esters or ethers of of natural and synthetic oils and waxes like PEG and/or PPG hydrogenated caster oils, among others. Non-limiting examples of colloids comprise at least one member of stearyl alcohols; behenyl alcohols; modified and unmodified gums like cargeenans, celluloses, carbomers; PVP; Hydrogenated and hydrolyzed waxes and/or oils; among others.

The SCC polymer can contain sufficient long chain groups that it will dissolve in the oil at a temperature above Tₚ. When the SCC polymer is used to thicken an oil or a mixture of oils which is free from water, the polymer generally contains at least about 50 %, normally at least about 60%, particularly at least about 70%, especially at about least 80%, of units comprising a linear radical containing about 10 to about 50 carbon atoms, and can contain up to 100% of such units. Particularly when the SCC polymer is used to thicken a water-in-oil or water-in-Silicone or multiple-phase emulsion, it may contain at least about 5%, usually at least 10%, of units derived from a monomer containing a functional group, such as at least one of hydroxyl, COOH and silicone functional groups and may contain higher amounts (e.g. up to 25% or 30wt%), provided that the SCC polymer will at least partially dissolve in the oil.

SCC polymers may consist of
(i) 70-99% by weight of repeating units derived from at least one n-alkyl acrylate or methacrylate ester in which the n-alkyl group contains 16 to 22 carbon atoms,
(ii) 1-50%, usually 15-25%, by weight of repeating units derived from at least one acrylate or methacrylate ester in which the ester group contains a hydroxyl-substituted alkyl group containing less than 12 carbon atoms, and
(iii) 0-30% by weight of repeating units derived from at least one acrylate or methacrylate ester in which the ester group contains an unsubstituted alkyl group containing less than 16 carbon atoms to lower the Tp.

SCC polymers may consist of
(i) 70-99% by weight of the repeating units derived from at least one n-alkyl acrylate or methacrylate ester in which the n-alkyl group contains 16 to 22 carbon atoms, and
(ii) 1-50%, typically 15-25%, by weight of the repeating units derived from at least one acrylate or methacrylate ester in which the ester group contains a hydroxyethyl, hydroxypropyl, or hydroxybutyl group.

SCC polymers may consist of
(i) 70-99% by weight of repeating units derived from at least one n-alkyl acrylate or methacrylate ester in which the n-alkyl group contains 16 to 22 carbon atoms,
(ii) 1-50%, usually 15-25%, by weight of repeating units derived from at least one silicon-containing monomer containing less than 12 carbon atoms, and
(iii) 0-30% by weight of repeating units derived from at least one acrylate or methacrylate ester in which the ester group contains an unsubstituted alkyl group containing less than 16 carbon atoms to lower the Tp.

SCC polymers may consist of
(i) 70-99% by weight of the repeating units derived from at least one n-alkyl acrylate or methacrylate ester in which the n-alkyl group contains 16 to 22 carbon atoms, and
(ii) 1-50%, typically 15-25%, by weight of the repeating units derived from at least one silicon-containing monomer comprising a member from at least one of butyl dimethicone methacrylate, trimethylsiloxy ethyl(meth)acrylate, polydimethyl-siloxane monmethyl methacrylate terminated, or 3 acryloxypropyl trimethoxysilane.

SCC polymers may consist of
(i) 70-99% by weight of repeating units derived from at least one n-alkyl acrylate or methacrylate ester in which the n-alkyl group contains 16 to 22 carbon atoms,
(ii) 1-50%, usually 15-25%, by weight of repeating units derived from at least one monomer containing a carboxylic group (COOH) containing less than 12 carbon atoms, and
(iii) 0-30% by weight of repeating units derived from at least one acrylate or methacrylate ester in which the ester group contains an unsubstituted alkyl group containing less than 16 carbon atoms to lower the Tp.

SCC polymers may consist of
(i) 70-99% by weight of the repeating units derived from at least one n-alkyl acrylate or methacrylate ester in which the n-alkyl group contains 16 to 22 carbon atoms, and
(ii) 1-50%, typically 15-25%, by weight of the repeating units derived from at least one monomer comprised from the list of acrylic acid, methacrylic acid, or maleic anhydride.

The molecular weight and other properties of the SCC polymer can be sufficiently controlled such that the polymer, after it has been dissolved in the oil, will crystallize in the oil when the heated mixture is cooled to the expected temperature of use, for example to a temperature 10-20 °C. below Tₚ, thus producing an opaque or clear mixture.

The Tₚ of the thickening polymer is normally 10-40 °C. above, particularly 10-30 °C. above, especially about 20 °C. above, the temperature at which the composition is to be used, which is generally 15-40 °C. It appears that the oil plasticizes the thickening polymer, so that its melting point in the composition is for example 5-20 °C. lower than Tₚ. It is therefore desirable that Tₚ is sufficiently above the temperature of use to ensure that the thickening polymer does not melt during use. Thus for compositions to be used at around 20-35 °C. the thickening polymer can have a Tₚ of above 40 °C, usually 40-70 °C. On the other hand, if the Tₚ of the thickening polymer is too far above the temperature of use, this can result in excessive crystallization and then precipitation of the polymer, thus reducing the thickening effect. It is useful, therefore, that Tₚ is not more than about 30 °C. above, usually not more than 20 °C. above, the temperature of use. Depending on the expected temperature of use, Tp may be from 0-150 °C, generally 10-100 °C., typically 40-70 °C., particularly 43-55 °C.

The amount of the polymeric thickener used may vary with the application. According to the present invention the functionalised SCC comprises poly C8-C22 alkylacrylates/ methacrylic acid crosspolymers and the SCC is present in in an amount of 10% or less by weight based on the weight of the oil. Smaller amounts such as about 2 to about 7% based on the weight of the oil in compositions which are free of water, and about 0.5 to about 5% based on the weight of composition in water-in-oil, water-in-silicone or multiple phase emulsions, are often effective. In one aspect of the invention, the amount of polymeric thickener is sufficient to form an emulsion (e.g., a water in oil, or an oil in water emulsion), having enhanced rheology and sensory properties. These properties can provide a sunscreen skin care emulsion having enhanced film building properties upon the skin and in turn increase the SPF of the sunscreen.

The amount of polymeric thickener may be sufficient to impart enhanced or controlled release of an active ingredient of a cosmetic composition, for example, by incorporating actives in the cosmetic media into amorphous crystalline matrix structure and releasing the actives in controlled manner at or near to body temperature. For example, the inventive composition can comprise an FSCC and/or SCC polymer containing antiperspirant or deodorant composition, or the composition can comprise one or more actives such as sunscreen actives or salicylic acid or any other cosmetic/personal care active used in emulsion formulations. Oils

The functionalized polymeric thickeners used in the present invention are effective with a broad range of oils such as at least one member selected from the group consisting of esters (C12-15 Alkyl benzoate), triglyceride (Caprylic/Caprylate triglyceride) hydrocarbons (mineral oil, sunflower oil) natural oils (jojoba oil, safflower oil) castor oil, among others. Suitable oils are also disclosed, for example, at column 3, line 37, to column 4, line 4, of U.S. Patent No. 5,736,125; For thickening silicone oils, it is useful to use an SCC polymer containing units derived from a monomer containing silicon, for example a block copolymer containing SCC blocks and polysiloxane blocks. SCC/polysiloxane polymers of this type are described for example in WO 93/07194 and WO 00/04787; Non-limiting examples of silicone oils can comprise at least one of dimethicone, pdms, organo silicone oils, dimethicones and cyclomethiconesln one aspect of the invention, the SCC polymers are employed as rhelogy modifiers (e.g., thickener) for at least one of dimethicone, cyclomethicone, and other low viscosity silicone oils..

Thickened oils with functionalized SCC provide a cosmetic formulation having unique aesthetic benefits (e.g., texture and feel). In some cases, the functionalized SCC and be combined with one or more SCC polymers. The ratio of SCC to functionalized SCC can range from about 0:1 to about 10:1. The following are non-limiting examples of cosmetic formulations containing thickened oils having such benefits:
A. Thickened Anhydrous Oils suitable for personal care applications (e.g. hair gels) comprising:
   Oils - about 50 to about 95wt%
   silicone oils - about 10 to about 95%
   Functionalized SCC or FSCC and
   SCC - about 1 to about 20wt.%
   Other additives/actives - about 0.1 to about 30%
B. Anhydrous Scalp Serum comprising:
   Oils - about 50 to about 95wt%
   Silicone oils - about 10 to about 95wt%
   Functionalized SCC or FSCC and
   SCC - about 1 to about 20wt%
   Other additives/actives - about 0.1 to about 30wt%
C. Anhydrous Sunscreen Stick or Gel comprising:
   Oils - about 50 to about 95wt%
   Silicone oils - about 10 to about 95wt%
   Functionalized SCC or FSCC and
   SCC - about 1 to about 20wt%
   Other additives/actives - about 0.1 to about 30wt%
D. Anhydrous Antiperspirant Deodorant (APDO) Stick or Gel comprising:
   Emollient - about 50 to about 95wt%
   Functionalized SCC or FSCC and
   SCC - about 1 to about 20wt%
   APDO actives - about 0.1 to about 30wt%
   Other Additives - about 1 to about 30wt%
E. Color Cosmetic such as blush, lipstick, among others comprising:
   Oil - about 50 to about 95 wt%
   Pigment - about 0.1 to about 30wt%
   Other additive - about 0.1 to about 10 wt.%
   Functionalized SCC or FSCC and
   SCC - about 1 to about 20wt%

If desired, conventional thickeners such as waxes like Carnauba wax, Bees wax, Candellila wax, among others can employed along with the FSCC and SCC polymers.

### Water-in-oil Emulsions

Water-in-oil emulsions are typically prepared by mixing together (1) a hot solution of the thickener in the oil and (2) the aqueous phase, the aqueous phase being at a temperature similar to the oil solution (e.g. not more than 10 °C. different); and then cooling the mixture while stirring. The ratio of the aqueous phase to the oil phase can be, for example, about 0.5:1 to about 9:1.

One aspect of the invention, relates to using the at least one functionalized SCC polymers, i.e. poly C₈-C₂₂ alkylacrylates/methacrylic acid crosspolymers, to prepare a water-in-oil emulsion. When employing SCC and functionalized SCC, the ratio of SCC to functionalized SCC can range from about 0:1 to about 10:1 The following are non-limiting examples of cosmetic formulations comprising water-in-oil emulsions:
A. Water in Silicone Oil Emulsions Containing Functionalized SCC and SCC
   i) Skin Moisturizer
      Water - about 50 to about 90wt%
      Silicone - about 1 to about 10wt%
      Emulsifier - about 0.5 to about 5wt%
      Emollient - about 5 to about 20wt%
      Functionalized SCC or FSCC and
      SCC - about 0.5 to about 3%
      Other Additives - about 0.1 to about 3wt%
   ii) Sunscreen
      Water - about 50 to about 90wt%
      Silicone - about 1 to about 10wt%
      Emulsifier - about 0.5 to about 5wt%
      Emollient - about 5 to about 20wt%
      Functionalized SCC or FSCC and
      SCC - about 0.5 to about 3wt%
      Sunscreen Active - about 1 to about 25wt%
      Other Additives - about 0.1 to about 3wt%
   iii) APDO
      Water - about 50 to about 90wt%
      Silicone - about 1 to about 10wt%
      Emulsifier - about 0.5 to about 5wt%
      Emollient - about 1 to about 20wt%
      Functionalized SCC or FSCC and
      SCC - about 0.5 to about 6wt%
      Antiperspirant active - about 1 to about 25wt%
      Other Additives - about 0.1 to about 5wt%
B. Water in oil Emulsion Containing Functionalized SCC
   i) Skin Moisturizer
      Water - about 50 to about 90wt%
      Emulsifier - about 0.5 to about 5wt%
      Emollient - about 5 to about 20wt%
      Functionalized SCC - about 0.5 to about 3wt%
      Other Additives - about 0.1 to about 3wt%
   ii) Sunscreen
      Water - about 50 to about 90wt% Emulsifier - about 0.5 to about 5wt% Emollient - about 5 to about 20wt% Functionalized SCC - about 0.5 to about 3wt% Sunscreen Active - about 1 to about 25wt% Other Additives - about 0.1 to about 3wt%

### Oil-in-water Emulsions

Oil-in-water emulsions are typically prepared by mixing together an oil phase into a water phase. The ratio of the Oil phase to the water phase can be, for example, about 0.1:1 to about 1:1. One aspect of the invention, relates to using at least one of functionalized SCC polymers , i.e. poly C₈-C₂₂ alkylacrylates/methacrylic acid crosspolymers, to prepare an oil-in-water emulsion. When employing functionalized SCC and SCC polymers, the ratio of SCC to functionalized SCC can range from about 0:1 to about 10:1. The following are non-limiting examples of cosmetic formulations comprising oil-in-water emulsions:
A. Oil in Water Emulsions
   i) Skin Moisturizer
      Water - about 50 to about 90wt%
      Emulsifier - about 0.5 to about 5wt%
      Emollient - about 1 to about 20wt%
      Functionalized SCC - about 0.5 to about 3wt%
      Other Additives - about 0.1 to about 3wt%
      If desired this Skin Moisturizer formulation can be modified by adding at least one SCC polymer.
   ii) Sunscreen
      Water - about 50 to about 90wt%
      Emulsifier - about 0.5 to about 5wt%
      Emollient - about 1 to about 20wt%
      Functionalized SCC - about 0.5 to about 3wt%
      Sunscreen Active - about 1 to about 25wt%
      Other Additives - about 0.1 to about 3wt%
      If desired this Sunscreen formulation can be modified by adding at least one SCC polymer
   iii) Mousse skin care or hair care styling
      Water - about 50 to about 90wt%
      Emulsifier - 0.5-1%
      Surfactant - 0.1-2%
      Functionalized SCC or FSCC and
      SCC - about 0.5 to about 1wt%
      Other Additives - about 0.1 to about 2wt%
      Solvent - about 1 to about 25wt%
      Propellant - about 6 to about 10wt%
B. Silicone in Water Emulsions
   Water - about 50 to about 90wt%
   Silicone - about 1 to about 5wt%
   Emulsifier - about 0.5 to about 5wt%
   Emollient - about 1 to about 20wt%
   Functionalized SCC or FSCC and
   SCC - about 0.5 to about 3wt%
   Other Additives - about 0.1 to about 3wt%

### Multiple Phase Emulsions

The previously described water-in-oil and oil-in-water emulsions can be used to prepare a multiple phase emulsion (e.g. water/oil/water, or oil/water/oil). Multiple phase emulsions can also be prepared by combining the previously described water-in-oil or oil-in-water emulsions with at least one FSCC, where the FSCC is in an emulsion or suspension form (e.g., emulsified Poly C12-22 AlkylAcrylates/Methacrylic Acid Crosspolymer).

### Alcohol Or Aqueous Systems

One aspect of the invention, relates to using at least one of functionalized SCC polymers, i.e. poly C₈-C₂₂ alkylacrylates/methacrylic acid crosspolymers, to prepare an alcohol or aqueous systems including emulsion. When employing functionalized SCC and SCC, the ratio of SCC to functionalized SCC can range from about 0:1 to about 10:1. These polymers can be used to thicken the alcohol or aqueous systems, as well as to provide benefits of film forming, controlled delivery or deposition of actives, and fragrance or volatiles retention. The following are non-limiting examples of cosmetic formulations comprising alcohol or aqueous systems:

Alcohol or Aqueous system with at least one of FSCC or at least one of FSCC and SCC polymers:
i). Mascara based upon FSCC,
ii). Coloring Shampoo
   Water - about 50 to about 90wt%
   Surfactant - about 2 to about 20wt%
   Foam booster -about 2 to about 20%
   FSCC or FSCC and SCC - about 0.5 to about 5 wt.%
   Other additives about 0.1 to about 10wt%,
iii). Hair Spray (aerosol and non-aerosol)
   Water - about 10 to about 90wt%
   FSCC or FSCC and SCC - about 0.5 to about 5wt%
   Ethanol or other solvents - about 33 to about 90wt%
   Optional Propellant for an aerosol - about 0 to about 50wt%
   Other additives - about 0.1 to about 2wt%,
iv). Shampoo
   Water - about 50 to about 90wt%
   Surfactant - about 2 to about 20wt%
   Foam booster - about 2 to about 20wt%
   FSCC or FSCC and SCC - about 0.1 to about 2 wt.%
   Other additives 0.1-10%,
v). Hair Styling products
   Water - about 10 to about90wt%
   FSCC or FSCC and SCC - about 0.5 to about5wt%
   Ethanol or other solvents- about 0 to about 10wt%
   Other additives - about 0.1 to about 10wt%,
vi). Body Wash / Hand cleansing product
   Water - about 50 to about90wt%
   Surfactant- about 2 to about 20wt%
   FSCC or FSCC and SCC - about 0.5 to about5wt%
   Foam booster - about 2 to about 20wt%
   Other additives about 0.1 to about 10wt%.

### Additives

If desired, one or more properties of a cosmetic composition can be controlled by adding a plasticizing compound to the composition. Examples of such compounds comprise at least one member selected from the group consisting of silicone based plasticiziers, natural or synthetic compounds (e.g., polysaccharides, natural or synthetic Gums, stabilizers, anionic and noionic associative thickener or rheology modifiers soluble in oil or water phase), other film forming polymers like polyurethanes, pyrolidines (e.g., polyvinylpyrolidine), among other compounds In one aspect of the invention, the additives comprise at least one member selected from the group consisting of preservatives, stabilizers (e.g., Xanthan Gum), humectant (e.g., at least one of Glycerine, MP Diol, Sorbitol, and Hexylene Glycol), antioxidant (e.g., Vitamins), rheology modifiers, fragrances, pigments, among other additives. In a further aspect of the invention, the additive can comprise at least one of surfactants and foam boosters. While any suitable surfactant and/or foam booster can be employed, examples of such comprise at least one member selected from the group consisting of sodium laureth sulfate, sodium lauryl sulfate, ammonium laureth sulfate, ammonium lauryl sulfate, cocamidopropyl betaine, among others. In a still further aspect of the invention, the additive can comprise at least one propellant and solvent such as at least one of isobutene, butane, dimethyl ether, ethanol, among others. The amount of additive typically ranges from about 0.1 to about 30 wt.% of the composition.

### Emulsifiers

If desired, one or more emulsifiers can be incorporated with the inventive composition. While any suitable emulsifier can be employed, examples of suitable emulsifiers comprise at least one member selected from the group consisting of glyceryl stearate, PEG-150 distearate, dlyceryl dilaurate, PEG-20 stearate, PEG-150 distearate, cetearyl alcohol (and) ceteareth-20, PEG-30 Dipolyhydroxystearate, among other compounds capable of forming or stabilizing an emulsion. The amount of emulsifier can range from about 0.5 to about 6 wt.% of the composition.

### Emollients

If desired, one or more emollients can be incorporated within the inventive composition. While any suitable emollient can be employed, examples of suitable emollients comprise at least one member selected from the group consisting of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart improved sensory or aesthetic properties of a personal care composition. The amount of emollient can range from about 1 to about 30 wt.% of the composition.

### Active Compounds

If desired, the inventive composition can be employed for delivering active compounds that interact with or protect skin or hair. Examples of such active compounds comprise at least one member selected from the group consisting of sunscreen active (zinc oxide, titanium dioxide, octinoxate, octocrylene, ethylhexyl Salicylate, oxybenzone, among others); Skin whitener (salicylic acid, among others); APDO actives (aluminum chlorohydrate, aluminum zirconium tetra chlorohydrex, among others), Vitamins (Tocopherol natural, Synthetic Tocopherol, Synthetic tocopherol acetate, Retinol, Retinyl palmitate, acetate, Provitamin B-5, Ascorbic acid, Sodium ascorbyl phosphate, Ascorbyl glucoside, Magnesium ascorbyl phosphate, among others); Polysaccharides (Hyaluronic acid, B-1,3-glucans, Chitosan, among others); Botanicals (Aloe vera, Green tea extract, Grape seed extract, Isoflavones, Chamomille/bisabolol, Fennel, Ginko, Ginseng, Guava, among others); Alpha Hydroxy Acids (Citric acid, Glycoloc acid, Lactic acid, among others), Sugar cane extracts; Coenzymes and Enzymes (Ubiquinone, Coenzyme Q10, among others); and cosmeceuticals, among other active ingredients

The inventive compositions maybe used to control the rate at which active compounds are provided. The rate can be controlled to be faster or slower than that possible without the inventive compositions. For example, the inventive compositions can be used for controlling the delivery rate of at least one of the following sunscreen actives (e.g., organic or inorganic), antiperspirant (e.g., aluminum APDO emulsion and stick), cosmeceutical compounds, antimicrobial compounds, among other compounds wherein controlled release is desirable. The compositions of the invention may be used for controlling the deposition or release of compounds such as relatively volatile compounds (e.g., fragrances), colorants (e.g., a coloring shampoo), among other compounds wherein it is desirable to control their deposition, delivery and/or release.

Certain aspects of the instant invention are illustrated by the following non-limiting Examples. The procedures used in the Examples to compare the effectiveness of the polymeric thickeners were as follows. In Examples 1-6, the thickener, 5 parts, was dissolved in hydrogenated polyisobutylene (HPIB, light oil), 95 parts, with stirring at 120 °C. The resulting solution was placed in an incubator at 20 °C. for 16 hours. The viscosity of the cooled product in centipoise was determined using a Brookfield DV-I+ digital viscometer with CP-51 spindle using a sample adapter which was thermostatically controlled, for example, to 25 °C. The viscosities were measured after four minutes at a speed of 2.5 rpm, i.e. after 10 revolutions. In Examples 7-12, the oil (as identified in Table 2), 14 parts, was heated to 80 °C., and the thickener, 0.75 parts, was dissolved therein. Water, 35 parts, containing MgSO₄.H₂O, 0.25 part, was heated to 80 °C. The oil and the water, both at 80 °C, were mixed together, and then cooled to 25 °C. with continued stirring. The product, a milky white water-in-oil emulsion, was left overnight, and its viscosity at 25 °C. was then measured using a Brookfield cone and plate viscometer. The viscosity was measured after 0.5, 1, 2 and 4 minutes, to assess the effect of shear on the emulsion.

The remaining Examples illustrate formulations of compositions incorporating functionalized SCC and/or SCC polymers. These Examples illustrate personal care formulations comprising skin care creams, lotions, and sun screens, body cleansing compositions (e.g., shower mouse), hair care (e.g., hair styling and conditioning), among other formulations.

### EXAMPLES

### Examples 1-12

Polymers and copolymers were made using the ingredients and amounts thereof shown in the Table below, using the following generalized method. To a resin kettle equipped with overhead stirrer and condenser was added 20% of the monomers and chain transfer agents. The mixture in the resin kettle was heated to 110 °C., and oxygen was removed from the system through nitrogen purge for about 30 min followed by addition of 20% of the starting initiator charge. After allowing sufficient time for any initial exotherm to abate, the remaining monomers, chain transfer agents and starting initiator were pumped into the reaction vessel over 60-90 min. The polymer mixture was allowed to continue reacting for 60 min followed by addition of the chase initiator and reaction for 60 min. The mixture was put under reduced pressure for 60 min to remove volatile residuals. The resulting polymers were generally yellow to white solids. The molecular weight, Tₚ, and viscosity of each sample were measured. The effectiveness of the polymers as thickeners was measured as described above, and the results are shown in Tables 1 and 2 below.

The following abbreviations are used in the Tables. ME = mercaptoethanol; MA = methacrylic acid; DMAEA = N,N-dimethylaminoethyl acrylate; HEA = 2-hydroxyethyl acrylate; TAPO = t-amylperoxy 2-ethylhexanoate sold by Witco as Esperox 570P, 75% active in liquid; TBPB = t-butylperoxybenzoate sold by Witco as Esperox 10; Estol is propylene glycol dicaprylate/caprate sold by Uniqema under the tradename Estol 1526; Min'l is mineral oil; and opq = opaque in appearance.

**TABLE 1**

| Example | 1 | 2 | 3 | 4 | 5 | 6 |
|---|---|---|---|---|---|---|
| C16A | | | | 95 | | |
| C18A | 80 | | 95 | | 100 | 85 |
| C22A | | 95 | | | | |
| HEA | 20 | | | | | |
| DMAEA | | | | | | 15 |
| MA | | 5 | 5 | 5 | | |
| ME | 0.34 | 0.17 | 0.17 | 0.17 | 0.17 | 0.1 |
| TAPO | 1.73 | 1.73 | 1.73 | 1.73 | 1.73 | 1.73 |
| TAPB | 0.67 | 0.67 | | | | |
| TBPB | | | 0.5 | 0.5 | 0.5 | 0.5 |
| Tp °C. | 48 | 67 | 47 | 39 | 50 | 45 |
| J/g | 56 | 99 | 57 | 64 | 73 | 60 |
| Mw | 236K | | 427K | 1,000K | 950K | |
| Mn | 52K | | 240K | 520K | 230K | |
| Bulk viscosity | 4,000 | 2,500 | 19,000 | 24,000 | 2,000 | 350 |
| Visc in HPIB | 12,600 opq | 164 Opq | 2600 opq | <50 clear | 5400 Opt | 6000 opq |

In Example 2, within the HPIB oil the polymer had a Tₚ above a desirable range, which resulted in excessive crystallinity and poor thickening under the test conditions. However, in other oils (e.g. isopropyl palmitate, or isopropyl myristate) sufficient plasticization of Tp for the polymer of Example 2 may still result in effective thickening. In Example 4, within the HPIB oil the polymer had a Tₚ below the desired range, and was ineffective as a thickener under the test conditions, because it did not crystallize on cooling. However, the polymer of Example 4 could still provide thickening or film forming benefits within an oil where little plasticization occurred (e.g. mineral oil, or ethanol).

**TABLE 2**

| Example | 7 | 8 | 9 | 10 | 11 | 12 |
|---|---|---|---|---|---|---|
| C18A | 80 | 80 | 80 | 100 | 100 | 100 |
| HEA | 20 | 20 | 20 | | | |
| ME | 0.34 | 0.34 | 0.34 | 0.17 | 0.17 | 0.17 |
| TAPO | 1.73 | 1.73 | 1.73 | 1.73 | 1.73 | 1.73 |
| TAPB | 0.67 | 0.67 | 0.67 | | | |
| TBPB | | | | 0.5 | 0.5 | 0.5 |
| Tp °C | 48 | 48 | 48 | 50 | 50 | 50 |
| J/g | 56 | 56 | 56 | 73 | 73 | 73 |
| Mw | 236K | 236K. | 236 K | 950 K. | 950 K. | 950 K. |
| Mn | 52K | 52 K. | 52 K. | 230 K. | 230 K. | 230 K. |
| Bulk viscosity | 4,000 | 4,000 | 4,000 | 2,000 | 2,000 | 2,000 |
| Oil | HPIB | Min'l | Estol | HPIB | Min'l | Estol |

| Viscosity after | | | | | | |
|---|---|---|---|---|---|---|
| 0.5 min | 35K | 37K | 45K | 29K | ** | ** |
| 1.0 min | 35K | 38K | 40K | 28K | | |
| 2.0 min | 41K | 38K | 34K | 25K | | |
| 4.0 min | 46K | 40K | 34K | 28K | | |

| | | | | | | |
|---|---|---|---|---|---|---|
| ** separated. | | | | | | |

### Example 13

Example 13 Sunscreen emulsion is formulated using following ingredients and procedure:

| Ingredients: | % w/w |
|---|---|
| Phase A: | |
| Deionized Water | q.s. |
| DOWICIL* 200 preservative | 0.10 |
| MP Diol | 1.50 |
| Xanthan Gum | 0.20 |
| Titanium dioxide | 5.0 |
| Dimethicone | 3.50 |
| C8-22 AlkylAcrylates/Methacrylic Acid Crosspolymer | 2.00 |
| | |

| Phase B: | |
|---|---|
| Octocrylene | 8.0 |
| Oxybenzone | 4.0 |
| Octinoxate | 10.0 |
| Caprylate/Caprate | 2.35 |
| Glyceryl Stearate | 1.60 |
| PEG-150 Distearate | 1.25 |
| Tridecyl Trimellitate | 0.75 |
| Stearic Acid, triple press | 0.65 |
| Emulsifying Wax, NF | 0.35 |
| Vitamin E | 0.10 |
| Vitamin A, Palmitate | 0.10 |
| | |

| Phase C: | |
|---|---|
| Deionized water | 1.00 |
| Imidazolidinyl Urea | 0.25 |
| | |

| Phase D: | |
|---|---|
| Fragrance | 0.3 |

### PROCEDURE

1. Combine water and glycol of Phase A at RT. Slowly sprinkle Carbopol into Phase A at RT with stirring. When uniform, begin heating to 70-75°C with stirring.
2. Combine Phase B; heat to 75-80°C; stir until uniform.
3. Slowly add Phase B to Phase A with homogenization at 70°C. When batch appears uniform; add Phase C with homogenization. When batch appears uniform, turn off heat. Switch to sweep at 60°C. Continue sweep throughout cool-down.
4. Add Phase D with stirring at 45°C.

Result: This body lotion is intended as an all-over body/face and hand moisturizer with SPF. The SCC Polymer acts as a viscosity modifier, imparts water resistance and lubricity while also yielding a non-oily feeling.
Comparative Example 13a: The preparation of example 13 is repeated using Poly C12-22 Alkyl Acrylates/Hydroxyethylacrylate Copolymer
Comparative Example 13b: The preparation of example 13 is repeated using C8-22 Alkyl Acrylates/Butyl Dimethicone Methacrylate Copolymer
Comparative Example 13c: The preparation of example 13 is repeated using Poly Acrylic Acid/C8-22 Alkyl Acrylate Copolymer

### Example 14

Sunscreen multi-phase emulsion is formulated using following ingredients and procedure:

| INGREDIENTS | %W/W |
|---|---|
| PHASE A | |
| Deionized Water | 62.80 |
| Disodium EDTA | 0.10 |
| Propylene Glycol | 2.00 |
| Xanthan Gum (Keltrol T) | 0.20 |
| Sorbitol 70% | 5.0 |
| | |

| PHASE B | |
|---|---|
| Octinoxate | 7.50 |
| Oxybenzone | 3.00 |
| Ethylhexyl Salicylate | 3.00 |
| Ethylhexyl Palmitate | 2.00 |
| Tridecyl Neopentanoate | 2.00 |
| Glyceryl Dilaurate | 1.50 |
| Mineral Oil | 6.00 |
| | |

| PHASE C | |
|---|---|
| Deionized Water | 5.00 |
| | |

| PHASE D | |
|---|---|
| Diazolidinyl Urea (and) lodopropynyl | |
| Butylcarbamate | 0.30 |
| Methylparben | 0.20 |
| Butylene Glycol | 1.00 |
| | |

| PHASE E | |
|---|---|
| C8-22 AlkylAcrylates/Methacrylic Acid Crosspolymer | 2.0 |

### PROCEDURE

1. Combine DI water Phase A with stirring at RT. When uniform, begin heating to 75-80°C with stirring.
2. Combine Phase B; heat to 75-80°C, stir until uniform.
3. Add Phase B to Phase A with homogenization at 75-80°C. When uniform, turn off heat.
4. Combine Phase C, mix until uniform. Slowly add Phase C to the batch with homogenization at 60°C.
5. When batch reaches 50°C, switch to sweep.
6. Individually add Phase D and E ingredients in order with sweep at 45°C, mixing well between additions.

Results: C8-22 AlkylAcrylates/Methacrylic Acid Crosspolymer provide very water-resistance SPF formulation
Comparative Example 14a: The preparation of example 14 is repeated using Poly C10-30 alkyl acrylate
Comparative Example 14b: The preparation of example 14 is repeated using Poly C12-22 Alkyl Acrylates/Hydroxyethylacrylate Copolymer
Comparative Example 14c: The preparation of example 14 is repeated using C8-22 Alkyl Acrylates/Butyl Dimethicone Methacrylate Copolymer
Comparative Example 14d: The preparation of example 14 is repeated using Poly Acrylic Acid/C8-22 Alkyl Acrylate Copolymer

### Example 15

Water-in-Silicone (w/Si) Sunscreen emulsion is formulated using following ingredients and procedure:

| INGREDIENTS | %W/W |
|---|---|
| PHASE A | |
| Dimethicone 200/100 | 5.00 |
| Mineral Oil | 1.00 |
| Ethylhexyl Palmitate | 1.00 |
| Octinoxate | 7.50 |
| Ethylhexyl Salicylate | 5.00 |
| Castor Oil | 0.50 |
| Beeswax | 0.50 |
| Polyethylene | 1.00 |
| PEG-30 Dipolyhydroxystearate | 2.00 |
| C8-22 AlkylAcrylates/Methacrylic Acid Crosspolymer | 0.5 |
| | |

| PHASE B | |
|---|---|
| Cyclopentasiloxane | 5.00 |
| Dimethicone | 5.00 |
| | |

| PHASE C | |
|---|---|
| Diazolidinyl Urea (and) lodopropynyl Butylcarbamate | 0.5 |
| | |

| PHASE D | |
|---|---|
| Sodium Chloride | 0.60 |
| Deionized Water | q.s |

### PROCEDURE

1. Combine ingredients in Phase A. Mix and heat to 85°C until uniform. Cool back to 70°C.
2. At 70°C add Phase B to Phase A. Mix and cool to 50°C.
3. Combine ingredients in Phase D. Mix and heat to 55°C.
4. With fast agitation, combine Phase D to Phases A and B. The incorporation should take at least 10 minutes.
5. Mix and cool to 35-40°C. Homogenize Phase C into batch when uniform.

Result: w/Si sunscreen emulsion is intended as an all-over body/face and hand SPF. The SCC Polymer acts as a viscosity modifier, imparts water resistance and lubricity while yielding a non-oily feeling.
Comparative Example 15a: The preparation of example 15 is repeated using Poly C10-30 alkyl acrylate
Comparative Example 15b: The preparation of example 15 is repeated using Poly C12-22 Alkyl Acrylates/Hydroxyethylacrylate Copolymer
Comparative Example 15c: The preparation of example 15 is repeated using C8-22 Alkyl Acrylates/Butyl Dimethicone Methacrylate Copolymer
Comparative Example 15d: The preparation of example 15 is repeated using Poly Acrylic Acid/C8-22 Alkyl Acrylate Copolymer

### Example 16

Water resistant sunscreen emulsion based on non-ionic emulsifiers is formulated using following ingredients and procedure:

| INGREDIENTS - | %W/W |
|---|---|
| PHASE A | |
| Deionized Water | q.s |
| Hexylene Glycol | 2.00 |
| Carbomer (Carbopol 980) | 0.20 |
| | |

| PHASE B | |
|---|---|
| Octinoxate | 7.50 |
| Oxibenzone | 3.00 |
| Ethylhexyl Salicylate | 3.00 |
| Ethylhexyl Palmitate | 6.00 |
| PEG-20 Stearate | 2.00 |
| Glyceryl Dilaureth | 3.00 |
| Myreth-3 myristate | 2.0 |
| | |

| PHASE C | |
|---|---|
| Deionized Water | 5.00 |
| Triethanolamine, 99% | 0.20 |
| | |

| PHASE D | |
|---|---|
| C8-22 AlkylAcrylates/Methacrylic Acid Crosspolymer | 1.5 |
| | |

| PHASE E | |
|---|---|
| Propylene Glycol (and) Diazolidinyl Urea (and) lodopropynyl | |
| Butylcarbamate | 0.60 |
| Phenoxyethanol (and) Methylparaben (and) Isobutylparaben (and) Butylparaben | 0.50 |

### PROCEDURE

1. Combine Phase A at RT. Slowly sprinkle Carbopol into Phase A at RT with stirring. When uniform, begin heating to 70-75°C with stirring.
2. Combine Phase B; heat to 75-80°C; stir until uniform.
3. Slowly add Phase B to Phase A with homogenization at 70°C. When batch appears uniform; add Phase C with homogenization. When batch appears uniform, turn off heat. Switch to sweep at 60°C. Continue sweep throughout cool-down.
4. Add Phase D with stirring at 65°C.
5. Add Phase E with stirring at 45°C.

Result: Water resistance Sunscreen emulsion is intended as an all-over body/face and hand moisturizer and SPF. The SCC Polymer acts as a viscosity modifier, impartswater resistance and lubricity while yielding anon-oily feeling.
Comparative Example 16a: The preparation of example 16 is repeated using Poly C10-30 alkyl acrylate
Comparative Example 16b: The preparation of example 16 is repeated using Poly C12-22 Alkyl Acrylates/Hydroxyethylacrylate Copolymer
Comparative Example 16c: The preparation of example 16 is repeated using C8-22 Alkyl Acrylates/Butyl Dimethicone Methacrylate Copolymer
Comparative Example 16d: The preparation of example 16 is repeated using Poly Acrylic Acid/C8-22 Alkyl Acrylate Copolymer

### Comparative Example 17

Example 17 illustrates personal care formulations using a silicone functional SCC polymer C8-22 Alkyl Acrylates/Butyl Dimethicone Methacrylate Copolymer. This polymer is an associative oil phase thickener designed to thicken a wide variety of silicone oils and silicone oil containing formulations including cyclomethicone, dimethicone, and various aliphatic and aromatic silicone oils. C8-22 Alkyl Acrylates/Butyl Dimethicone Methacrylate Copolymer has a built-in temperature switch (48°C) to allow change in properties at trigger temperature.

### Experimental Details:

Preparation of thickened oil: C8-22 Alkyl Acrylates/Butyl Dimethicone Methacrylate Copolymer and oil (10/90 by wt) were heated together to 80°C with agitation ensuring dissolution, followed by cooling in a 25°C bath for at least 16 hours before measuring viscosity.

Preparation of emulsions: C8-22 Alkyl Acrylates/Butyl Dimethicone Methacrylate Copolymer (3.0g) was dissolved in oil (27.0g) at 80°C. DI water containing 0.5% magnesium sulfate (70g) was heated to 80°C and added to the stirred hot oil phase. The water/oil mixture was agitated with a homogenizer during cooling. The formulation was allowed to sit in a 25°C bath for at least 16 hours prior to testing viscosity.

Viscosity was evaluated at 25°C using a Brookfield DV-I+ viscometer equipped with cone and plate spindle CP51 at 2.5 rpm. Data points were taken at 30, 60, 120 and 240 seconds and reported in cps.

The SCC polymers are able to thicken different oils in cosmetic media examples of oils are esters like C12-15 alkyl benzoate, triglycerides such as Caprylic/caprylate triglyceride, hydrocarbon like mineral oil, sunflower oil, natural oils like jojoba oil, safflower oil, caster oil, organo-silicones, dimethicones, and cyclomethicones.

### Comparative Example 18

Water-in-Silicone Moisturizing Cream is formulated using following ingredients and procedure:

| Ingredients: | % w/w |
|---|---|
| Phase A: | |
| Stearic acid, triple press | 1.20 |
| Cetearyl alcohol (and) ceteareth-20 | 1.85 |
| PEG-30 dipolyhdroxysterate | 1.85 |
| Mineral oil | 1.35 |
| Cyclometnicone | 5.00 |
| Cetyl alcohol | 1.10 |
| PEG 150 distearate | 1.00 |
| Dimethicone | 4.60 |
| Emulsifying Wax | 0.60 |
| Tridecyl Trimellitate | 0.50 |
| Mango Butter | 0.50 |
| C8-22 Alkyl Acrylates/Butyl Dimethicone | |
| Methacrylate Copolymer | 2.00 |
| | |

| Phase B: | |
|---|---|
| Deionized Water | q.s. |
| Xanthan gum | 0.25 |
| MP Diol | 5.00 |
| Imidazolidinyl Urea | 0.25 |
| | |

| Phase C: | |
|---|---|
| Fragrance | 0.3 |

### PROCEDURE:

1. Combine Phase A ingredients with stirring and heating to 70-75°C.
2. Combine Phase B at RT when uniform heat to 75-80°C; stir until uniform.
3. Slowly add Phase B to Phase A with homogenization at 65°C. When batch appears uniform, turn off heat. Switch to sweep at 60°C. Continue sweep throughout cool-down. Add Phase C at 40°C.

Results: Cream is formulated to soften and moisturize skin. The lubricity of C8-22 Alkyl Acrylates/Butyl Dimethicone Methacrylate Copolymer helps provide relatively easy application qualities and after feel. The skin is left with an emollient, non-oily, velvety feeling. The SCC Polymer acts as a viscosity modifier, imparts water resistance and lubricity while yielding a non-oily feeling.
Comparative Example 18a: The preparation of example 18 is repeated using Poly C10-30 alkyl acrylate
Comparative Example 18b: The preparation of example 18 is repeated using Poly C12-22 Alkyl Acrylates/Hydroxyethylacrylate Copolymer
Example 18c: The preparation of example 18 is repeated using C8-22 AlkylAcrylates/Methacrylic Acid Crosspolymer except this polymer is added in phase C.
Comparative Example 18d: The preparation of example 18 is repeated using Poly Acrylic Acid/C8-22 Alkyl Acrylate Copolymer

### Comparative Example 19

Water-in-oil SPF and Moisturizing Lotion is formulated using following ingredients and procedure:

| Ingredients: | % w/w |
|---|---|
| Phase A: | |
| Octocrylene | 8.0 |
| Oxybenzone | 4.0 |
| Octinoxate | 10.0 |
| Caprylate/Caprate triglyceride | 2.35 |
| Glyceryl Stearate (and) | |
| PEG-100 sterate | 1.60 |
| PEG-150 Distearate | 1.25 |
| Tridecyl Trimellitate | 0.75 |
| Stearic Acid, triple press | 0.65 |
| Emulsifying Wax, NF | 0.35 |
| Vitamin E | 0.10 |
| Vitamin A, Palmitate | 0.10 |
| C8-22 Alkyl Acrylates/Butyl Dimethicone | |
| | |
| Methacrylate Copolymer | 3.00 |

| Phase B: | |
|---|---|
| Deionized Water | q.s. |
| MP Diol | 1.50 |
| Xanthan Gum | 0.20 |
| Titanium dioxide | 5.0 |
| Dow Corning 193 | 3.50 |
| Imidazolidinyl Urea | 0.25 |
| | |

| Phase C: | |
|---|---|
| Fragrance | 0.3 |

### PROCEDURE

1. Combine water and glycol of Phase B at RT. Slowly sprinkle Xanthan Gum into Phase B at RT with stirring. When uniform, begin heating to 70-75°C with stirring.
2. Combine Phase A; heat to 75-80°C; stir until uniform.
3. Slowly add Phase B to Phase A with homogenization at 60°C. When batch appears uniform; add Phase C with homogenization and stirring at 45°C.

Results: Emollient skin feel lotion is intended as an all-over body/face and hand moisturizer and SPF. The C8-22 Alkyl Acrylates/Butyl Dimethicone Methacrylate Copolymer acts as a viscosity modifier, imparts water resistance and lubricity while yielding a non-oily feeling.
Comparative Example 19a: The preparation of example 19 is repeated using Poly C12-22 Alkyl Acrylates/Hydroxyethylacrylate Copolymer
Example 19b: The preparation of example 19 is repeated using C8-22 AlkylAcrylates/Methacrylic Acid Crosspolymer except this polymer is added in phase C.
Comparative Example 19c: The preparation of example 19 is repeated using Poly Acrylic Acid/C8-22 Alkyl Acrylate Copolymer

### Comparative Example 20

Water-in-oil Skin care emulsion is formulated using following ingredients and procedure:

| INGREDIENTS | %W/W |
|---|---|
| PHASE A | |
| Castor Oil | 3.00 |
| Ethylhexyl Palmitate | 2.00 |
| Tridecyl Neopentanoate | 2.00 |
| Glyceryl Oleate | 1.50 |
| PEG-30 dipolyhydroxystearate | 6.00 |
| C8-22 Alkyl Acrylates/Butyl Dimethicone | |
| Methacrylate Copolymer | 1.00 |
| | |

| PHASE B | |
|---|---|
| Deionized Water | 62.80 |
| Disodium EDTA | 0.10 |
| Propylene Glycol | 2.00 |
| Xanthan Gum | 0.20 |
| Sorbitol 70% | 5.0 |
| | |

| PHASE C | |
|---|---|
| Deionized Water | 5.00 |
| | |

| PHASE D | |
|---|---|
| Diazolidinyl Urea (and) lodopropynyl | |
| Butylcarbamate | 0.30 |
| METHYLPARABEN | 0.20 |
| Butylene Glycol | 1.00 |

### PROCEDURE

1. Combine Phase B with stirring and begin heating to 75-80°C with stirring.
2. Combine Phase A; heat to 75-80°C, stir until uniform.
3. Add Phase B to Phase A with homogenization at 65°C. When uniform, turn off heat.
4. Combine Phase C, mix until uniform. Slowly add Phase C to the batch with homogenization at 60°C.
5. When batch reaches 50°C, switch to sweep.
6. Individually add Phase D ingredients in order with sweep at 45°C, mixing well between additions.

Results: Lotion with smooth, elegant feel/touch and easy spreadibility
Comparative Example 20a: The preparation of example 20 is repeated using Poly C12-22 Alkyl Acrylates/Hydroxyethylacrylate Copolymer
Example 20b: The preparation of example 20 is repeated using C8-22 AlkylAcrylates/Methacrylic Acid Crosspolymer except this polymer is added in phase C.
Comparative Example 20c: The preparation of example 20 is repeated using Poly Acrylic Acid/C8-22 Alkyl Acrylate Copolymer

### Comparative Example 21

Water-in-Silicone (w/Si) Sunscreen is formulated using following ingredients and procedure:

| INGREDIENTS | %W/W |
|---|---|
| PHASE A | |
| Dimethicone 200/100 | 5.00 |
| Mineral Oil | 1.00 |
| Ethylhexyl Palmitate | 1.00 |
| Octinoxate | 7.50 |
| Ethylhexyl Salicylate | 5.00 |
| Castor Oil | 0.50 |
| Beeswax | 0.50 |
| Polyethylene | 1.00 |
| PEG-30 Dipolyhydroxystearate | 2.00 |
| C8-22 Alkyl Acrylates/Butyl Dimethicone | |
| Methacrylate Copolymer | 2.00 |
| | |

| PHASE B | |
|---|---|
| Cyclopentasiloxane | 5.00 |
| Dimethicone | 5.00 |
| | |

| PHASE C | |
|---|---|
| Diazolidinyl Urea (and) lodopropynyl Butylcarbamate | 0.5 |
| | |

| PHASE D | |
|---|---|
| Sodium Chloride | 0.60 |
| Deionized Water | q.s |

### PROCEDURE

1. Combine ingredients in Phase A. Mix and heat to 80°C until uniform. Cool back to 70°C.
2. At 70°C add Phase B to Phase A. Mix and cool to 50°C.
3. Combine ingredients in Phase D. Mix and heat to 55°C.
4. With fast agitation, combine Phase D to Phases A and B. The incorporation should take at least 10 minutes.
5. Mix and cool to 35-40°C. Homogenize Phase C and D into batch when uniform.

Results: Water-in-Silicone sunscreen emulsion with easy pick-up
Comparative Example 21a: The preparation of example 21 is repeated using Poly C10-30 alkyl acrylate
Comparative Example 21b: The preparation of example 21 is repeated using Poly C12-22 Alkyl Acrylates/Hydroxyethylacrylate Copolymer except this polymer is added in phase C.
Example 21c: The preparation of example 21 is repeated using C8-22 AlkylAcrylates/Methacrylic Acid Crosspolymer
Comparative Example 21d: The preparation of example 21 is repeated using Poly Acrylic Acid/C8-22 Alkyl Acrylate Copolymer

### Comparative Example 22

Water resistance sunscreen lotion is formulated using following ingredients and procedure:

| INGREDIENTS - | %W/W |
|---|---|
| PHASE A | |
| Deionized Water | q.s |
| Hexylene Glycol | 2.00 |
| Carbomer (Carbopol 980) | 0.20 |
| | |

| PHASE B | |
|---|---|
| Octinoxate | 7.50 |
| Oxibenzone | 3.00 |
| Ethylhexyl Salicylate | 3.00 |
| Ethylhexyl Palmitate | 6.00 |
| PEG-20 Stearate | 2.00 |
| Glyceryl Dilaureth | 3.00 |
| Myreth-3 myristate | 2.0 |
| C8-22 Alkyl Acrylates/Butyl Dimethicone | |
| Methacrylate Copolymer | 0.50 |
| | |

| PHASE C | |
|---|---|
| Deionized Water | 5.00 |
| Triethanolamine, 99% | 0.20 |
| | |

| PHASE D | |
|---|---|
| Propylene Glycol (and) Diazolidinyl Urea (and) lodopropynyl | |
| Butylcarbamate | 0.60 |
| Phenoxyethanol (and) Methylparaben (and) Isobutylparaben | |
| (and) Butylparaben | 0.50 |

### PROCEDURE

1. Combine water and glycol of Phase A at RT. Slowly sprinkle Carbopol into Phase A at RT with stirring. When uniform, begin heating to 70-75°C with stirring.
2. Combine Phase B; heat to 75-80°C; stir until uniform.
3. Slowly add Phase B to Phase A with homogenization at 70°C. When batch appears uniform; add Phase C with homogenization. When batch appears uniform, turn off heat. Switch to sweep at 60°C. Continue sweep throughout cool-down.
4. Add Phase D with stirring at 45°C and stir to RT.

Results: Sunscreen lotion with velvety feel due to use of C8-22 Alkyl Acrylates/Butyl Dimethicone Methacrylate Copolymer.
Comparative Example 22a: The preparation of example 22 is repeated using Poly C12-22 Alkyl Acrylates/Hydroxyethylacrylate Copolymer
Example 22b: The preparation of example 22 is repeated using C8-22 AlkylAcrylates/Methacrylic Acid Crosspolymer
Comparative Example 22c: The preparation of example 22 is repeated using Poly Acrylic Acid/C8-22 Alkyl Acrylate Copolymer

### Comparative Example 23

Shower Mousse is formulated using following ingredients and procedure:

| Ingredient | %W/W |
|---|---|
| Phase A | |
| Deionized Water | q.s. to 100 |
| Sodium Laureth Sulfate | 20.00 |
| Cocamidopropyl Betaine | 2.00 |
| Propylene Glycol | 2.00 |
| Glycerine | 1.00 |
| Benzophenone-4 | 0.05 |
| Propylene Glycol (and) Diazolidinyl | |
| Urea (and) Methylparaben (and) | |
| Propylparaben | 0.40 |
| | |

| Phase B | |
|---|---|
| Octinoxate | 7.5 |
| C8-22 Alkyl Acrylates/Butyl Dimethicone | |
| Methacrylate Copolymer | 2.00 |
| | |

| Phase C | |
|---|---|
| Propellant A-31 Isobutane | 6.00 |

### PROCEDURE

Combine Phase A, ingredients into water with moderate agitation and increase temperature to 65°C and mix phase B in separate vessel and increase temperature to 55°C. Add phase B slowly to phase C while mixing. Bring phase A B mixture to room temperature. Fill cans with concentrate and charge propellant with Phase C.
Results: Shower Mousse that leaves the skin feeling soft and smooth due to use of C8-22 Alkyl Acrylates/Butyl Dimethicone Methacrylate Copolymer.
Comparative Example 23a: The preparation of example 23 is repeated using Poly C10-30 alkyl acrylate
Comparative Example 23b: The preparation of example 23 is repeated using Poly C12-22 Alkyl Acrylates/Hydroxyethylacrylate Copolymer
Example 23c: The preparation of example 23 is repeated using C8-22 AlkylAcrylates/Methacrylic Acid Crosspolymer except this polymer is added in phase A
Comparative Example 23d: The preparation of example 23 is repeated using Poly Acrylic Acid/C8-22 Alkyl Acrylate Copolymer

### Example 24

Hair Combing Cream is formulated using following ingredients and procedure:

| Ingredient | %W/W |
|---|---|
| Phase A | |
| Cetearyl Alcohol | 1.80 |
| Ceteareth-20 | 0.10 |
| Behentrimonium Chloride | 0.44 |
| Amodimethicone (and) | |
| Trideceth-12(and) | |
| Cetrimonium Chloride | 3.50 |
| C8-22 Alkyl Acrylates/Butyl Dimethicone | |
| Methacrylate Copolymer | 2.00 |
| | |

| Phase B | |
|---|---|
| DI Water | q.s to 100 |
| Cetrimonium Chloride | 0.30 |
| | |

| Phase C | |
|---|---|
| DMDM Hydantoin | 0.20 |
| Phenoxyethanol (and) Methylparaben | |
| (and) Ethylparaben (and) Butylparaben | |
| (and) Propylparaben (and) | |
| Isobutylparaben | 0.15 |
| | |

| Phase D | |
|---|---|
| Citric Acid (10% solution) or | |
| Sodium hydroxide (10% solution) | q.s to pH 4.0-5.0 |

### PROCEDURE

Combine Phase A with mixing and heat to 80°C. Combine Phase B in a separate vessel, combine the components of Phase B and heat to 80°C. Add Phase B to Phase A with agitation. Maintain the temperature and add Phase C. Continue mixing and cool to 45°C. Use Phase D to adjust the pH if needed.
Results: In addition to conditioning normal hair, the combing cream provides curl definition, reducing the volume and making it easier to control. C8-22 Alkyl Acrylates/Butyl Dimethicone Methacrylate Copolymer maintains the natural shape of curls while offering a soft appearance along with temperature control restyling benefits. C8-22 Alkyl Acrylates/Butyl Dimethicone Methacrylate Copolymer provides a smooth and silky feel to the hair and improves the emulsion stability.
Comparative Example 24a: The preparation of example 24 is repeated using Poly C10-30 alkyl acrylate
Comparative Example 24b: The preparation of example 24 is repeated using Poly C12-22 Alkyl Acrylates/Hydroxyethylacrylate Copolymer
Example 24c: The preparation of example 24 is repeated using C8-22 AlkylAcrylates/Methacrylic Acid Crosspolymer except this polymer is added in phase B
Comparative Example 24d: The preparation of example 24 is repeated using Poly Acrylic Acid/C8-22 Alkyl Acrylate Copolymer

### Comparative Example 25

Conditioning Cream is formulated using following ingredients and procedure:

| Ingredients | %W/W |
|---|---|
| Deionized Water | q.s. to 100 |
| Xanthan Gum | 1.50 |
| DMDM Hydantoin | 0.5 |
| | |
| Mineral Oil | 3.00 |
| C8-22 Alkyl Acrylates/Butyl Dimethicone | |
| Methacrylate Copolymer | 0.5 |
| Candililla Wax | 1.5 |

### PROCEDURE:

Charge deionized water into vessel. Using good agitation, slowly sift in the Xanthan Gum. When completely hydrated, add the preservative and the oil. Maintain good agitation and add the C8-22 Alkyl Acrylates/Butyl Dimethicone Methacrylate Copolymer and increase temperature to 75°C. Once dispersed, homogenize if necessary adjust the pH to 4.8 to 5.0 if needed.
Results: This cream-gel formulation provides enhanced hold, high humidity curl retention, and shine to the hair. C8-22 Alkyl Acrylates/Butyl Dimethicone Methacrylate Copolymer polymer gives thickening and hold.
Comparative Example 25a: The preparation of example 25 is repeated using Poly C10-30 alkyl acrylate
Comparative Example 25b: The preparation of example 25 is repeated using Poly C12-22 Alkyl Acrylates/Hydroxyethylacrylate Copolymer
Example 25c: The preparation of example 25 is repeated using C8-22 AlkylAcrylates/Methacrylic Acid Crosspolymer
Comparative Example 25d: The preparation of example 25 is repeated using Poly Acrylic Acid/C8-22 Alkyl Acrylate Copolymer

### Comparative Example 26

Extra Conditioning Spray Gel is formulated using following ingredients and procedure:

| Ingredients | %/ W/W |
|---|---|
| Phase A | |
| Polyquaternium-10 | 1.00 |
| Deionized Water (Aqua) | q.s. to 100 |
| | |

| Phase B | |
|---|---|
| C8-22 Alkyl Acrylates/Butyl Dimethicone | |
| Methacrylate Copolymer | 1.00 |
| Propylene Glycol | 0.1 |
| Glycerin | 0.10 |
| Germaben II Propylene Glycol (and) Diazolidinyl Urea (and) Methylparaben (and) Propylparaben | 0.20 |

### PROCEDURE:

Prepare Phase A into warm water and increase the temperature to 75°C with agitation until completely dispersed. Once solution is homogenous, add Phase B to Phase A when temperature of both phases are at 75°C and mix well and cool to room temperature.

### VALVE SPECIFICATIONS

Pump Type: Seaquist Euromist HV
Body: 190 mcl output
Insert: 16 x 10 deep

Results: This alcohol free gel contains C8-22 Alkyl Acrylates/Butyl Dimethicone Copolymer, which provides hold and conditioning with a natural feel. It also act as a rheology modifier will offer good spray aesthetics.
Comparative Example 26a: The preparation of example 26 is repeated using Poly C10-30 alkyl acrylate
Comparative Example 26b: The preparation of example 26 is repeated using Poly C12-22 Alkyl Acrylates/Hydroxyethylacrylate Copolymer
Example 26c: The preparation of example 26 is repeated using C8-22 AlkylAcrylates/Methacrylic Acid Crosspolymer except this polymer is added in phase B
Comparative Example 26d: The preparation of example 26 is repeated using Poly Acrylic Acid/C8-22 Alkyl Acrylate Copolymer

### Comparative Example 27

55% volatile organic compound (VOC) Pump Hair Spray is formulated using following ingredients and procedure:

| Ingredients | % W/W |
|---|---|
| C8-22 Alkyl Acrylates/Butyl Dimethicone | |
| | |
| Methacrylate Copolymer | 5.00 |
| AMP 95 Aminomethyl Propanol | to pH 8.5 to 8.9 |
| Deionized Water | q.s. to 100 |
| *SD Alcohol 40 | 55.00 |

| | |
|---|---|
| *Substitution of SD Alcohol 40 with 64.17% SD Alcohol 23A (190 proof and containing 7.3% acetone denaturant) would result in improved tack and dry times while maintaining 55% VOC compliance. | |

### PREPARATION:

Dissolve AMP in SD Alcohol 40 and water. While maintaining proper agitation, slowly add Intelimer 1221. Mix until homogenous. Filter and fill containers.

### VALVING

Supplier: SeaquistPerfect
Type: EM Classic
Body: 160 mcl Output
Actuator: 0.010" x 0.010" Deep

Results: This formulation provides desirable spray aesthetics, hold, and good humidity resistance.
Comparative Example 27a: The preparation of example 27 is repeated using Poly C10-30 alkyl acrylate
Comparative Example 27b: The preparation of example 27 is repeated using Poly C12-22 Alkyl Acrylates/Hydroxyethylacrylate Copolymer
Example 27c: The preparation of example 27 is repeated using C8-22 AlkylAcrylates/Methacrylic Acid Crosspolymer except this polymer is added in phase B
Comparative Example 27d: The preparation of example 27 is repeated using Poly Acrylic Acid/C8-22 Alkyl Acrylate Copolymer

### Comparative Example 28

80% VOC Pump Hair Spray is formulated using following ingredients and procedure:

| Ingredients | % W/W |
|---|---|
| C8-22 Alkyl Acrylates/Butyl Dimethicone | |
| | |
| Methacrylate Copolymer | 5.00 |
| AMP 95 Aminomethyl Propanol | to pH 8.5 to 8.9 |
| Deionized Water | q.s. |
| SD Alcohol 40 | 80.00 |

### PREPARATION:

Dissolve AMP in SD Alcohol 40 and water. While maintaining proper agitation, slowly add Intelimer 1221. Mix until homogenous. Filter and fill containers.
Results: This formulation provides excellent spray aesthetics and elegant feel. Comparative Example 28a: The preparation of example 28 is repeated using Poly C10-30 alkyl acrylate
Comparative Example 28b: The preparation of example 28 is repeated using Poly C12-22 Alkyl Acrylates/Hydroxyethylacrylate Copolymer
Example 28c: The preparation of example 28 is repeated using C8-22 AlkylAcrylates/Methacrylic Acid Crosspolymer except this polymer is added in phase B
Comparative Example 28d: The preparation of example 28 is repeated using Poly Acrylic Acid/C8-22 Alkyl Acrylate Copolymer

### Comparative Example 29

Styling Hair Gel with is formulated using following ingredients and procedure:

| Ingredients | % W/W |
|---|---|
| Phase A | |
| Carbomer 940 | 0.25 |
| Deionized Water | q.s. |
| | |

| Phase B | |
|---|---|
| C8-22 Alkyl Acrylates/Butyl Dimethicone | |
| | |
| Methacrylate Copolymer | 3.00 |
| Dimethicone 200 5cst fluid | 0.50 |
| Mineral oil | 0.10 |
| Hydrolyzed Wheat Protein (and) Hydrolyzed Wheat Starch | 0.10 |
| Propylene Glycol and Diazolidinyl Urea and Methylparaben and Propylparaben | 1.00 |

| Phase C | |
|---|---|
| Triethanolamine 99% | to adjust to pH 8 |

### PREPARATION

Prepare Phase A into warm water (∼50°C) with agitation until completely dispersed. Once solution is homogenous, add Phase B and mix well. Add the Triethanolamine drop wise to bring the pH up to around 8. When the pH is close to 8, the formulation will thicken and become clear.
Comparative Example 29 a: The preparation of example 29 is repeated using Poly C10-30 alkyl acrylate
Comparative Example 29 b: The preparation of example 29 is repeated using Poly C12-22 Alkyl Acrylates/Hydroxyethylacrylate Copolymer
Example 29 c: The preparation of example 29 is repeated using C8-22 AlkylAcrylates/Methacrylic Acid Crosspolymer except this polymer is added in phase B
Comparative Example 29d: The preparation of example 29 is repeated using Poly Acrylic Acid/C8-22 Alkyl Acrylate Copolymer

### Comparative Example 30

Example 30 illustrates personal care formulations using a carboxy functional SCC polymer.

### INCI NAME:

Acrylic Acid/C12-22 Alkyl Acrylates Copolymer

### Features:

Associative oil phase thickener designed to thicken a wide variety of oils including mineral oils, hydrogenated polyisobutylene (HPIB), vegetable oils, oil esters, triglycerides, etc. Acrylic Acid/C12-22 Alkyl Acrylates Copolymer has a built-in temperature switch (65°C) to allow change in properties at the trigger temperature. Particularly effective in polar oils and for suspending particles

### Experimental Details:

Preparation of thickened oil: Acrylic Acid/C12-22 Alkyl Acrylates Copolymer and oil (10/90 by wt) were heated together to 80°C with agitation ensuring dissolution, followed by cooling in a 25°C bath for at least 16 hours before measuring viscosity.
Preparation of emulsions: Acrylic Acid/C12-22 Alkyl Acrylates Copolymer (3.0g) was dissolved in oil (27.0g) at 80°C. DI water containing 0.5% magnesium sulfate (70g) was heated to 80°C and added to the stirred hot oil phase. The water/oil mixture was agitated with a homogenizer during cooling. The formulation was allowed to sit in a 25°C bath for at least 16 hours prior to testing viscosity.

Viscosity was evaluated at 25°C using a Brookfield DV-I+ viscometer equipped with cone and plate spindle CP51 at 2.5 rpm. Data points were taken at 30, 60, 120 and 240 seconds and reported in cps.

### Comparative Example 31

Example 31 illustrates personal care formulations using a hydroxyl functional SCC polymer

### INCI NAME:

C12-22 Alkyl Acrylates/Hydroxyethylacrylate Copolymer

### Features:

Associative oil phase thickener designed to thicken a wide variety of oils including mineral oils, hydrogenated polyisobutylene (HPIB), vegetable oils, oil esters, triglycerides, etc. C12-22 Alkyl Acrylates/Hydroxyethylacrylate Copolymer has a built-in temperature switch (65°C) to allow change in properties at the trigger temperature. Particularly effective in polar oils.

### Experimental Details:

Preparation of thickened oil: C12-22 Alkyl Acrylates/Hydroxyethylacrylate Copolymer and oil (10/90 by wt) were heated together to 80°C with agitation ensuring dissolution, followed by cooling in a 25°C bath for at least 16 hours before measuring viscosity.

### Preparation of emulsions: C12-22 Alkyl Acrylates/Hydroxyethylacrylate Copolymer

(3.0g) was dissolved in oil (27.0g) at 80°C. DI water containing 0.5% magnesium sulfate (70g) was heated to 80°C and added to the stirred hot oil phase. The water/oil mixture was agitated with a homogenizer during cooling. The formulation was allowed to sit in a 25°C bath for at least 16 hours prior to testing viscosity.

### Comparative Example 32

Mild Deep Cleansing Shampoo with is formulated using following ingredients and procedure:

| Ingredient | %W/W |
|---|---|
| Phase A | |
| Deionized Water | q.s. |
| Stepan Mild BSB(42% active) | |
| Water, PEG-80 Sorbitan Laurate, Cocamidopropyl Betaine, Sodium | |
| Trideceth Sulfate, Disodium Lauroamphodiacetate, PEG-150 | |
| Distearate, Sodium Laureth-13, Carboxylate, Quaternium-15, Tetrasodium EDTA, DMDM Hydantoin, Citric Acid | 35.71 |
| Standapol A (29% active) Ammonium Lauryl Sulfate | 5.17 |
| | |

| Phase B | |
|---|---|
| Mineral oil | 5.0 |
| Intelimer Polymer | 1.0 |
| | |

| Phase C | |
|---|---|
| Glydant DMDM Hydantoin | 0.40 |

### PROCEDURE

Combine phase A and increase temperature to 75°C. Dissolve Intelimer Polymer in mineral oil by increasing temperature to 75°C. Add phase B to phase A slowly. Add all other ingredients one at a time allowing the system to become homogeneous before next addition. Add phase C at 45°C. Adjust the pH to 5.5 - 6.0
Comparative Example 32a: The preparation of example 32 is repeated using Poly C10-30 alkyl acrylate
Comparative Example 32b: The preparation of example 32 is repeated using C8-22 Alkyl Acrylates/Butyl Dimethicone Methacrylate Copolymer
Example 32c: The preparation of example 32 is repeated using C8-22 AlkylAcrylates/Methacrylic Acid Crosspolymer except this polymer is added in phase B
Comparative Example 32d: The preparation of example 32 is repeated using Poly Acrylic Acid/C8-22 Alkyl Acrylate Copolymer

### Comparative Example 33

Temporary coloring shampoo is formulated using following ingredients and procedure:

| Ingredients | % W/W |
|---|---|
| Phase A | |
| Arianor Dye (any color) | 1.00 Warner |
| Jenkinson | |
| Distilled Water | q.s. to 100 |
| | |

| Phase B | |
|---|---|
| Miranol C2M-SF Disodium | |
| Cocoamphodipropionate | 8.00 |
| Incromine Oxide L (30%) Lauramine Oxide | 14.00 |
| Promidium CO PPG-2 Hydroxyethyl Cocamide | 3.00 |
| | |

| Phase C | |
|---|---|
| Mineral Oil | 4.00 |
| C8-22 Alkyl Acrylates/Butyl Dimethicone | |
| Methacrylate Copolymer | 1.00 |
| | |

| Phase D | |
|---|---|
| Distilled Water | 28.00 |
| Glydant (55%) DMDM Hydantoin | 0.50 |

### PREPARATION

Combine Phase A. Mix well. Combine Phase B with overhead agitation. Mix well. Combine Phase C and increase the temperature of all phases separately to 75C. Add Phase C to Phase B. Mix well. Combine Phase D. Mix well. Add Phase D to Phase BC. Mix well. Add Phase A to Phases BCD making sure to completely disperse dye.. Mix well for ten minutes.
Results: C12-22 Alkyl Acrylates/Hydroxyethylacrylate Copolymer builds viscosity in this mild shampoo also provides moderate conditioning and assist in delivering color pigment effectively.
Comparative Example 33a: The preparation of example 33 is repeated using Poly C10-30 alkyl acrylate
Comparative Example 33b: The preparation of example 33 is repeated using C8-22 Alkyl Acrylates/Butyl Dimethicone Methacrylate Copolymer
Example 33c: The preparation of example 33 is repeated using C8-22 AlkylAcrylates/Methacrylic Acid Crosspolymer except this polymer is added in phase B
Comparative Example 33d: The preparation of example 33 is repeated using Poly Acrylic Acid/C8-22 Alkyl Acrylate Copolymer

### Comparative Example 34

Hot Blusher is formulated using following ingredients and procedure:

| Ingredients | % W/W |
|---|---|
| Permethyl 102A Isoeicosane | 45.10 |
| C12-22 Alkyl Acrylates/ | |
| Hydroxyethylacrylate Copolymer | 4.00 |
| Isopropyl Myristate | 4.00 |
| C-7055 Yellow Iron Oxide | 0.50 |
| C-7051 Red Iron Oxide | 1.00 |
| C-7133 Black Iron Oxide | 0.20 |
| Propylparaben | 0.20 |
| 09985 AW TiO2 Titanium Dioxide | 9.00 |
| Mineral oil | q.s. to 100 |

### PROCEDURE

Charge all ingredients into main kettle, heat to 82°C. Mix for 20-30 minutes or until uniform. Check for color dispersion via draw-down. Pour into appropriate pans at 77-79°C.

Results: C12-22 Alkyl Acrylates/Hydroxyethylacrylate Copolymer is an oil soluble polymer that allows high load of emollients while still obtaining an elegant matte finish.

Due to its oil solubility this copolymer can be used in any color cosmetic application without any need of different waxes that are commonly used in color cosmetic like carnauba wax, candela wax, bees wax that including lip sticks and mascara. If desired, however, the above mentioned polymer can be combined with such waxes.
Comparative Example 34a: The preparation of example 34 is repeated using Poly C10-30 alkyl acrylate
Comparative Example 34b: The preparation of example 34is repeated using C8-22 Alkyl Acrylates/Butyl Dimethicone Methacrylate Copolymer
Example 34c: The preparation of example 34 is repeated using C8-22 AlkylAcrylates/Methacrylic Acid Crosspolymer except this polymer is added in phase B
Comparative Example 34d: The preparation of example 34 is repeated using Poly Acrylic Acid/C8-22 Alkyl Acrylate Copolymer

### Comparative Example 35

Water-in-oil emulsion was formulated using following ingredients and procedure:

| Ingredients | % w/w |
|---|---|
| Phase A | |
| C12-22 Alkyl Acrylates/Hydroxyethylacrylate Copolymer | 1.00 |
| Isopropyl Palmitate | 4.00 |
| Isopropyl Myristate | 4.00 |
| Mineral Oil USP | 1.00 |
| PEG-30 Dipoly Hydroxy Stearate | 0.50 |
| Bis-PEG/PPG-14/14 Dimethicone | 2.00 |
| Cyclomethicone | 11.5 |

| Phase B | |
|---|---|
| DI Water | 69.5 |
| MgSO4 | 0.50 |
| Glycerin | 5.00 |
| Propylene Glycol (and) Diazolidinyl Urea(and) Methyl Paraben (and) Propyl Paraben | 1.00 |
| Total | 100 |

### PROCEDURE

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes switched to sweep at 60°C. Continue sweep throughout cool-down process.

Results: This Example demonstrates the ability to modify the rheology and oil phase thickener in Cosmetic/Personal Care formulations:
Comparative Example 35a: The preparation of example 35 was repeated using Poly C10-30 alkyl acrylate
Comparative Example 35b: The preparation of example 35 was repeated using C8-22 Alkyl Acrylates/Butyl Dimethicone Methacrylate Copolymer
Example 35c: The preparation of example 35 was repeated using C8-22 AlkylAcrylates/Methacrylic Acid Crosspolymer except this polymer is added in phase B
Comparative Example 35d: The preparation of example 35 was repeated using Poly Acrylic Acid/C8-22 Alkyl Acrylate Copolymer

### Comparative Example 36

Example 36 illustrates anhydrous cosmetic/personal care formulations using the following ingredients and procedure:

| | |
|---|---|
| Anhydrous gel 1 | |

| INCI Name | %w/w |
|---|---|
| C12-22 Alkyl Acrylates/Hydroxyethylacrylate | |
| Copolymer | 5.00 |
| Mineral Oil | 25.25 |
| Cococaprylate/Caprate | 20.2 |
| Caprylic Capric Triglyceride | 14.3 |
| Octyldodecanol | 17.7 |
| Cyclohexasiloxane (and) Cyclopentasiloxane | 6.5 |
| Sunflower Seed oil | 3.3 |
| Octinoxate | 7.5 |
| Vitamin E | 0.25 |
| | 100 |

| Ingredients (anhydrous gel 2) | INCI Name |
|---|---|
| Mineral Oil Drakeol 7 | 96.0 |
| PEG-4 | 1.00 |
| C12-22 Alkyl Acrylates/Hydroxyethylacrylate Copolymer | 3.00 |
| Total | 100.0 |

| Ingredients (anhydrous gel 3) | INCI Name |
|---|---|
| Dimethicone | 56.0 |
| Cyclomethicone | 39.0 |
| C12-22 Alkyl Acrylates/Hydroxyethylacrylate Copolymer | 5.0 |
| Total | 100.0 |

| Ingredients (anhydrous sunscreen gel 4) | INCI Name |
|---|---|
| Isopropyl Palmitate | 52.0 |
| Octocrylene | 4.00 |
| Octinoxate | 7.50 |
| Cyclomethicone | 31.5 |
| C12-22 Alkyl Acrylates/ Hydroxyethylacrylate Copolymer | 5.0 |
| Total | 100.0 |

### PROCEDURE FOR ANHYDROUS GEL FORMULATIONS:

All the ingredients were combined and temperature was increased slowly to 75°C let all the ingredients mix until homogeneous. Cool down to room temperature. Thick anhydrous gel or stick was formed and thickness vary depend upon use level.

Results: This Example demonstrates the ability to thicken oil phase in anhydrous Cosmetic/Personal Care formulations:
Comparative Example 36a: The preparation of examples 36 were repeated using Poly C10-30 alkyl acrylate
Comparative Example 36b: The preparation of examples 36 were repeated using C8-22 Alkyl Acrylates/Butyl Dimethicone Methacrylate Copolymer
Example 36c: The preparation of examples 36 were repeated using C8-22 AlkylAcrylates/Methacrylic Acid Crosspolymer except this polymer is added in phase B
Comparative Example 36d: The preparation of examples 36 were repeated using Poly Acrylic Acid/C8-22 Alkyl Acrylate Copolymer

### Comparative Example 37

Example 37 illustrates Water-in-Silicone emulsions for use in cosmetic/personal care compositions.

| Ingredients | % w/w |
|---|---|
| Phase A | |
| C12-22 Alkyl Acrylates/Hydroxyethylacrylate Copolymer | 1.5 |
| | |
| Cyclopentasiloxane and PEG/PPG-18/18 Dimethicone | 10.0 |
| | |
| Cyclopentasiloxane | 16.0 |
| | |
| C12-14 Alkyl Benzoate | 0.5 |

| Phase B | |
|---|---|
| Distilled Water | q.s. to 100 |
| Glycerin | 5.0 |
| Sodium Chloride | 2.0 |
| Propylene Glycol (and) Diazolidinyl Urea(and) Methyl Paraben (and) Propyl Paraben | 1.0 |
| Total | 100 |

### PROCEDURE

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes switched to sweep at 60°C. Continue sweep throughout cool-down process.

Results: This Example demonstrates the ability to thicken oil phase in Cosmetic/Personal Care formulations:
Comparative Example 37a: The preparation of example 37 was repeated using Poly C10-30 alkyl acrylate
Comparative Example 37b: The preparation of example 37 was repeated using C8-22 Alkyl Acrylates/Butyl Dimethicone Methacrylate Copolymer
Example 37c: The preparation of example 37 was repeated using C8-22 AlkylAcrylates/Methacrylic Acid Crosspolymer except this polymer is added in phase B
Comparative Example 37d: The preparation of example 37 was repeated using Poly Acrylic Acid/C8-22 Alkyl Acrylate Copolymer

### Comparative Example 38

Sunscreen cosmetic/personal care formulations were formulated using following ingredients and procedure:

| In-Vitro SPF 29 | |
|---|---|
| Sunscreen | %w/w |
| INCI Name | |
| Phase A | |
| Octinoxate | 7.5 |
| Benzophenone-3 | 6.0 |
| Octyl Salicylate | 5.0 |
| Mineral Oil | 3.0 |
| C12-15 alkyl benzoate | 5.0 |
| C12-22 Alkyl Acrylates/ Hydroxyethylacrylate Copolymer | 3.0 |
| Steareth-2 | 2.5 |
| Steareth-21 | 2.5 |

| Phase B | |
|---|---|
| Xanthan Gum | 0.2 |
| DI Water | q.s. to 100 |
| MP Diol | 5.0 |

| Phase C | |
|---|---|
| Panthenol | 0.25 |

| Phase D | |
|---|---|
| Propylene Glycol (and) Diazolidinyl Urea(and) Methyl Paraben (and) Propyl Paraben | 1.00 |
| Total | 100.0 |

**Sunscreen Formulation:**

| | |
|---|---|
| Ingredient | |

| Phase A | % w/w |
|---|---|
| DI Water | q.s to 100 |
| Hydroxy Propyl | |
| Methylcellulose | 0.1 |
| Xanthan Gum | 0.2 |

| Phase B | |
|---|---|
| Octinoxate | 7 |
| Oxibenzone | 6 |
| Octyl Salicylate | 5 |
| C12-15 Alkyl Benzoate | 5 |
| Potassium Cetyl Phosphate | 0.5 |
| Sorbitan Oleate | 0.1 |
| C12-22 Alkyl Acrylates/Hydroxyethylacrylate Copolymer | 2 |
| Caprylyl/Caprylic Triglyceride | 10 |
| Phase C | |
| Panthenol | 0.3 |

| Phase D | |
|---|---|
| Propylene Glycol (and) Diazolidinyl Urea(and) Methyl Paraben (and) Propyl Paraben | 1.0 |
| | |
| Vitamin E | 0.2 |
| Total | 100 |

### PROCEDURE:

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes. Phase C was added during homogenization process, switched to sweep at 60°C. Continue sweep throughout cool-down process.

Results: Cosmetic/personal care formulations having enhanced SPF and resistance to water rub off/removal:
Comparative Example 38a: The preparation of examples 38 were repeated using C8-22 Alkyl Acrylates/Butyl Dimethicone Methacrylate Copolymer
Example 38b: The preparation of examples 38 were repeated using C8-22 AlkylAcrylates/Methacrylic Acid Crosspolymer except this polymer is added in water phase
Comparative Example 38c: The preparation of examples 38 were repeated using Poly Acrylic Acid/C8-22 Alkyl Acrylate Copolymer
Comparative Example 38d: The preparation of examples 38 were repeated using Poly C10-30 alkyl acrylate Copolymer

### Comparative Example 39

Moisturizing emulsion was formulated using following ingredients and procedure:

| Phase A | |
|---|---|
| | q.s. to |
| DI Water | 100 |
| Xanthan Gum | 0.2 |
| Allantoin | 0.25 |

| Phase B | |
|---|---|
| | |
| Glyceryl Stearate and PEG-100 Stearate | 2.6 |
| Polyoxyethylene 40 Stearate | 2.5 |
| Hydrogenated Castor oil | 2.0 |
| Cetearyl Alcohol Ceteareth-20 | 2.5 |
| Isopropyl Myristate | 15.5 |
| Cocoyl Capric caprylate | 5.0 |
| C12-22 Alkyl Acrylates/Hydroxyethylacrylate Copolymer | 2.0 |

| Phase C | |
|---|---|
| Panthenol | 0.25 |

| Phase D | |
|---|---|
| | |
| Diazolidinyl Urea(and) Methyl Paraben (and) Propyl Paraben | 1.0 |
| Total | 100 |

### PROCEDURE:

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes. Phase C was added during homogenization process, switched to sweep at 60°C. Continue sweep throughout cool-down process.

Results: Example 39 illustrates a cosmetic/personal care composition having enhanced Trans Epidermal Water Loss (TEWL) prevention or occlusivity.
Comparative Example 39 a: The preparation of example 39 was repeated using C8-22 Alkyl Acrylates/Butyl Dimethicone Methacrylate Copolymer
Example 39 b: The preparation of example 39 was repeated using C8-22 AlkylAcrylates/Methacrylic Acid Crosspolymer except this polymer is added in phase B
Comparative Example 39 c: The preparation of example 39 was repeated using Poly Acrylic Acid/C8-22 Alkyl Acrylate Copolymer
Comparative Example 39 d: The preparation of example 39 was repeated using Poly C10-30 alkyl acrylate.

### Comparative Example 40

Moisturizing Water-in-Silicone Cream was formulated using following ingredients and procedure:

| Ingredients | % w/w |
|---|---|
| Phase A | |
| C12-22 Alkyl Acrylates/Hydroxyethylacryl ate Copolymer | 3.00 |
| | |
| Cyclopentasiloxane and PEG/PPG-18/18 Dimethicone | 10.0 |
| | |
| Cyclopentasiloxane | 16.0 |
| | |
| C12-14 Alkyl Benzoate | 0.5 |

| Phase B | |
|---|---|
| Distilled Water | 62.5 |
| Glycerin | 5.00 |
| Sodium Chloride | 2.00 |
| Propylene Glycol (and) Diazolidinyl Urea(and) Methyl Paraben (and) Propyl Paraben | 1.00 |
| Total | 100 |

### PROCEDURE

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes switched to sweep at 60°C. Continue sweep throughout cool-down process.

Comparative Example 40a: The preparation of example 40 was repeated using C8-22 Alkyl Acrylates/Butyl Dimethicone Methacrylate Copolymer
Comparative Example 40b: The preparation of example 40 was repeated using C8-22 AlkylAcrylates/Methacrylic Acid Crosspolymer except this polymer is added in phase B
Example 40c: The preparation of example 40 was repeated using Poly Acrylic Acid/C8-22 Alkyl Acrylate Copolymer
Comparative Example 40d: The preparation of example 40 was repeated using Poly C10-30 alkyl acrylate

### Comparative Example 41

Antiperspirant Deodorant (APDO) stick was formulated using following ingredients and procedure:

| INCI Name | %w/w |
|---|---|
| Stearyl Alcohol | 28.0 |
| PPG-14 Butyl Ether | 28.5 |
| | |
| Polydimethylcyclosiloxanes | 5.1 |
| C12-15 Alkyl Benzoate | 5.3 |
| Hydrogenated Cater Oil | 5.0 |
| Aluminum Zirconium Tetra Chlorohydrex Gly (80% active) (18.48 active) | 23.1 |
| C12-22 Alkyl Acrylates/Hydroxyethylacrylate Copolymer | 5.0 |
| | |
| Total | 100 |

### PROCEDURE

All the ingredients were combined and temperature was increased slowly to 75°C let all the ingredients mix until homogeneous. Cool down to room temperature. Thick anhydrous gel or stick was formed and thickness vary depend upon use level.
Results: Example 41 illustrates a cosmetic/personal care formulation having controlled release properties that can be employed as an Antiperspirant/Deodorant
Comparative Example 41a: The preparation of example 41 was repeated using C8-22 Alkyl Acrylates/Butyl Dimethicone Methacrylate Copolymer
Example 41b: The preparation of example 41 was repeated using C8-22 AlkylAcrylates/Methacrylic Acid Crosspolymer
Comparative Example 41c: The preparation of example 41 was repeated using Poly Acrylic Acid/C8-22 Alkyl Acrylate Copolymer
Comparative Example 41d: The preparation of example 41 was repeated using Poly C10-30 alkyl acrylate

### Comparative Example 42

Water-in-oil Antiperspirant Deodorant (APDO) emulsion was formulated using following ingredients and procedure:

| Ingredients | % w/w |
|---|---|
| Phase A | |
| C12-22 Alkyl Acrylates/Hydroxyethylacrylate Copolymer | 5.0 |
| Mineral Oil USP | 4.0 |
| Cyclopentasiloxane | 11.5 |
| PEG-30 Dipoly Hydroxy Stearate | 2.0 |

| Phase B | |
|---|---|
| Glycerine | 5.0 |
| D.I. Water | 32.0 |
| MgSO4 | 0.5 |
| Aluminum Chlorohydrate(20% active in water) | 40.0 |
| Total | 100 |

Results: Example 42 illustrates a cosmetic/personal care formulation having controlled release properties that can be employed as an Antiperspirant/Deodorant
Comparative Example 42a: The preparation of example 42 was repeated using C8-22 Alkyl Acrylates/Butyl Dimethicone Methacrylate Copolymer
Example 42b: The preparation of example 42 was repeated using C8-22 AlkylAcrylates/Methacrylic Acid Crosspolymer except this polymer is added in water phase
Comparative Example 42c: The preparation of example 42 was repeated using Poly Acrylic Acid/C8-22 Alkyl Acrylate Copolymer
Comparative Example 42d: The preparation of example 42 was repeated using Poly C10-30 alkyl acrylate

### Comparative Example 43

Lipstick was formulated using following ingredients and procedure:

| INCI Designation | %W/W |
|---|---|
| Isopropyl Palmitate | 35.8 |
| Lanolin oil | 2.5 |
| Isononyl Isononanoate | 3.1 |
| Octyldodecanol | 3.2 |
| PEG-4 diheptanoate | 6.4 |
| See below | 38.8 |
| Isopropylparaben (and) Isobutylparaben (and) | 0.2 |
| Butylparaben | |
| C12-22 Alkyl Acrylates/Hydroxyethylacrylate Copolymer | 10.0 |
| | |

| Pigment Pre-blend | %W/W |
|---|---|
| Ricinus Communis [castor] seed oil | 96.81 |
| Black iron oxide[and] ricinus communis [castor] seed oil | 0.44 |
| D&C Red #27 Aluminum Lake [and] ricinus communis [castor] seed oil | 1.5 |
| D&C Red #7 Calcium Lake [and] ricinus communis [castor] seed oil | 1.0 |
| Iron Oxide [and] ricinus communis [castor] seed oil) | 0.25 |

### PROCEDURE

Combine Phase A ingredients in the order above in a main vessel and begin heating to 75-80°C while stirring. Continue to stir until all ingredients are completely dispersed and uniform. Once uniform, pour batch into a proper mold at 70-72°C.

Results: Example 43 illustrates a cosmetic/personal care formulation having Lipstick with thickening provided by C12-22 Alkyl Acrylates/Hydroxyethylacrylate Copolymer.

Due to its oil solubility this copolymer can be used in any color cosmetic application without any need of different waxes that are commonly used in color cosmetic like carnauba wax, candela wax, bees wax that including lip sticks and mascara. If desired, however, the above mentioned polymer can be combined with such waxes.
Comparative Example 43a: The preparation of example 43 was repeated using C8-22 Alkyl Acrylates/Butyl Dimethicone Methacrylate Copolymer
Example 43b: The preparation of example 43 was repeated using C8-22 AlkylAcrylates/Methacrylic Acid Crosspolymer except this polymer is added in phase B
Comparative Example 43c: The preparation of example 43 was repeated using Poly Acrylic Acid/C8-22 Alkyl Acrylate Copolymer
Comparative Example 43d: The preparation of example 43 was repeated using Poly C10-30 alkyl acrylate

## Claims

1. A personal care composition comprising
(1) water,
(2) at least one oil,
(3) at least one functionalised side chain crystalline (SCC) polymer wherein said polymer comprises poly C₈-C₂₂ alkylacrylates/methacrylic acid crosspolymers wherein the amount of SCC is sufficient to form an emulsion and wherein the amount of SCC is 10 % or less by weight based on the weight of the oil; and
(4) at least one emulsifier.

2. The composition of Claim 1 wherein the oil comprises at least one silicone oil.

3. The composition of Claim 1 wherein the composition comprises an oil-in-water emulsion.

4. The composition of Claim 1 wherein the composition comprises a water-in-oil emulsion.

5. The composition of Claim 1 further comprising at least one active compound.

6. The composition of Claim 1 further comprising at least one surfactant.

7. The composition of Claim 1 further comprising at least one propellant.

8. The composition of Claim 1 further comprising at least one solvent.

9. The composition of Claim 5 wherein said active compound comprises at least one member selected from the group consisting of zinc oxide, titanium dioxide, octinoxate, ctocrylene, ethylhexyl salicylate, oxybenzone, salicylic acid, aluminum chlorohydrate, aluminum zirconium tetra chlorohydrex, vitamin E and vitamin A, tocopherol natural, synthetic tocopherol, synthetic tocopherol acetate, retinol, retinyl palmitate, acetate, provitamin B-5, ascorbic acid, sodium ascorbyl phosphate, ascorbyl glucoside, magnesium ascorbyl phosphate, polysaccharides, hyaluronic acid, B-1,3-glucans, chitosan, botanicals, aloe vera, green tea extract, grape seed extract, isoflavones, chamomille/bisabolol, fennel, ginko, ginseng, guava, alpha hydroxy acids, citric acid, glycolic acid, lactic acid, sugar cane extracts, coenzymes and enzymes, ubiquinone, and coenzyme Q 10.

10. The composition of Claim 1 wherein said emulsifier comprises at least one member selected from the group consisting of glyceryl stearate, PEG-150 distearate, glyceryl dilaurate, PEG-20 stearate, PEG-150 distearate, cetearyl alcohol (and) ceteareth-20, and PEG-30 Dipolyhydroxystearate.

11. The composition of Claim 1 wherein the composition comprises at least one member selected from the group consisting of cosmetics, color cosmetics, toiletries, cleansers, blush, lipsticks, antiperspirant deodorants, deodorants, nail varnishes, sunscreens, hand protective products, anti-aging or skin renewal products, night renewal products, body milks, facial products, protective day care products, moisturizer, shampoo, hair gel, mascara, and hair sprays.

12. The composition of Claim 1 wherein the amount of emulsifier is 0.5 to 6 weight percent of the composition.

13. The composition of Claim 1, wherein the composition further comprises at least one member selected from the group consisting of triglycerides, C₁₂-C₁₅ alkyl benzoates, mineral oil and natural oils.

14. The composition of Claim 2 wherein the silicone oil comprises at least one of dimethicone and cyclomethicone.

15. The composition of Claim 1 further comprising at least one member selected from the group consisting of glycerine, hexylene glycol, natural gums and polysaccharides.

16. A method for preparing a personal care composition comprising combining the ingredients of Claim 1.

## Patentansprüche

1. Körperpflegezusammensetzung, umfassend:
(1) Wasser,
(2) mindestens ein Öl;
(3) mindestens ein funktionalisiertes seitenkettenkristallines (SCC)-Polymer, wobei das Polymer Poly-C₈-C₂₂-alkylacrylate/Methacrylsäure-Kreuzpolymere umfasst, wobei die Menge an SCC ausreicht, um eine Emulsion zu bilden, und wobei die Menge an SCC 10 Gew.% oder weniger beträgt, bezogen auf das Gewicht des Öls; und
(4) mindestens einen Emulgator.

2. Zusammensetzung nach Anspruch 1, wobei das Öl mindestens ein Silikonöl umfasst.

3. Zusammensetzung nach Anspruch 1, wobei die Zusammensetzung eine Öl-in-Wasser-Emulsion umfasst.

4. Zusammensetzung nach Anspruch 1, wobei die Zusammensetzung eine Wasser-in-Öl-Emulsion umfasst.

5. Zusammensetzung nach Anspruch 1, die des Weiteren mindestens eine Wirkverbindung umfasst.

6. Zusammensetzung nach Anspruch 1, die des Weiteren mindestens ein Tensid umfasst.

7. Zusammensetzung nach Anspruch 1, die des Weiteren mindestens ein Treibmittel umfasst.

8. Zusammensetzung nach Anspruch 1, die des Weiteren mindestens ein Lösungsmittel umfasst.

9. Zusammensetzung nach Anspruch 5, wobei die Wirkverbindung mindestens ein Mitglied ausgewählt aus der Gruppe bestehend aus Zinkoxid, Titandioxid, Octinoxat, Octocrylen, Ethylhexylsalicylat, Oxybenzon, Salicylsäure, Aluminiumhydroxychlorid, Aluminiumzirconiumtetrachlorohydrex, Vitamin E und Vitamin A, natürlichem Tocopherol, synthetischem Tocopherol, synthetischem Tocopherolacetat, Retinol, Retinylpalmitat, Acetat, Provitamin B-5, Ascorbinsäure, Natriumascorbylphosphat, Ascorbylglucosid, Magnesiumascorbylphosphat, Polysacchariden, Hyaluronsäure, β-1,3-Glucanen, Chitosan, Pflanzeninhaltstoffen, Aloe vera, Grünteeextrakt, Traubenkernextrakt, Isoflavonen, Kamille/Bisabolol, Fenchel, Ginko, Ginseng, Guave, alpha-Hydroxysäuren, Citronensäure, Glykolsäure, Milchsäure, Zuckerrohrextrakten, Coenzymen und Enzymen, Ubiquinon und Coenzym Q 10 umfasst.

10. Zusammensetzung nach Anspruch 1, wobei der Emulgator mindestens ein Mitglied ausgewählt aus der Gruppe bestehend aus Glycerylstearat, PEG-150-Distearat, Glyceryldilaurat, PEG-20-Stearat, PEG-150-Distearat, Cetearylalkohol (und) Ceteareth-20 sowie PEG-30-Dipolyhydroxystearat umfasst.

11. Zusammensetzung nach Anspruch 1, wobei die Zusammensetzung mindestens ein Mitglied ausgewählt aus der Gruppe bestehend aus Kosmetika, Farbkosmetika, Hygieneartikeln, Reinigungsmitteln, Rouge, Lippenstiften, Antiperspirant-Deodorantien, Deodorantien, Nagellacken, Sonnenschutzmitteln, Handschutzprodukten, Anti-Aging- oder Hauterneuerungsprodukten, Erneuerungsprodukten für die Nacht, Körpermilchen, Gesichtsprodukten, schützenden Tagespflegeprodukten, Feuchthaltepräparaten, Shampoo, Haargel, Mascara und Haarsprays umfasst.

12. Zusammensetzung nach Anspruch 1, wobei die Menge an Emulgator 0,5 bis 6 Gew.% der Zusammensetzung beträgt.

13. Zusammensetzung nach Anspruch 1, wobei die Zusammensetzung ferner mindestens ein Mitglied ausgewählt aus der Gruppe bestehend aus Triglyceriden, C₁₂-C₁₅-Alkylbenzoaten, Mineralöl und natürlichen Ölen umfasst.

14. Zusammensetzung nach Anspruch 2, wobei das Silikonöl mindestens eines von Dimethicon und Cyclomethicon umfasst.

15. Zusammensetzung nach Anspruch 1, ferner umfassend mindestens ein Element ausgewählt aus der Gruppe bestehend aus Glycerin, Hexylenglykol, natürlichen Gummis und Polysacchariden.

16. Verfahren zur Herstellung einer Körperpflegezusammensetzung, umfassend Kombinieren der Bestandteile von Anspruch 1.

## Revendications

1. Composition de soin personnel, comprenant
(1) de l'eau ;
(2) au moins une huile ;
(3) au moins un polymère cristallin à chaîne latérale (SCC) fonctionnalisé, où ledit polymère comprenant des polymères croisés de polyacrylates de C₈-C₂₂-alkyle/acide méthacrylique, où la quantité de SCC est suffisante pour former une émulsion et où la quantité de SCC est de 10% ou moins en poids, sur la base du poids de l'huile ; et
(4) au moins un émulsifiant.

2. Composition selon la revendication 1, où l'huile comprend au moins une huile de silicone.

3. Composition selon la revendication 1, où la composition comprend une émulsion huile-dans-eau.

4. Composition selon la revendication 1, où la composition comprend une émulsion eau-dans-huile.

5. Composition selon la revendication 1, comprenant en outre au moins un composé actif.

6. Composition selon la revendication 1, comprenant en outre au moins un agent tensioactif.

7. Composition selon la revendication 1, comprenant en outre au moins un propulseur.

8. Composition selon la revendication 1, comprenant en outre au moins un solvant.

9. Composition selon la revendication 5, où ledit composé actif comprend au moins un élément choisi dans le groupe constitué par l'oxyde de zinc, le dioxyde de titane, l'octinoxate, l'octocrylène, le salicylate d'éthylhexyle, l'oxybenzone, l'acide salicylique, le chlorhydrate d'aluminium, le tétrachlorohydrex d'aluminium et de zirconium, la vitamine E et la vitamine A, le tocophérol naturel, le tocophérol synthétique, l'acétate de tocophérol synthétique, l'acétate, le palmitate de rétinyle, la provitamine B5, l'acide ascorbique, le phosphate d'ascorbyle sodique, l'ascorbyl glucoside, le phosphate d'ascorbyle et de magnésium, les polysaccharides, l'acide hyaluronique, les β-1,3-glucanes, le chitosane, les extraits de plantes, l'aloès, l'extrait de thé vert, l'extrait de pépins de raisin, les isoflavones, la camomille/bisabolol, le fenouil, le ginkgo, le ginseng, la goyave, les acides alpha-hydroxylés, l'acide citrique, l'acide glycolique, l'acide lactique, les extraits de canne à sucre, les coenzymes et les enzymes, l'ubiquinone, et le coenzyme Q10.

10. Composition selon la revendication 1, où ledit émulsifiant comprend au moins un élément choisi dans le groupe constitué par le stéarate de glycérol, le distéarate de PEG-150, le dilaurate de glycérol, le stéarate de PEG-20, le distéarate de PEG-150, l'alcool cétéarylique (et) cétéareth-20, et le dipolyhydroxy-stéarate de PEG-30.

11. Composition selon la revendication 1, où la composition comprend au moins un élément choisi dans le groupe constitué par les produits cosmétiques, les produits cosmétiques de couleur, les articles de toilette, les lotions nettoyantes, le fard à joues, les rouges à lèvres, les déodorants antitranspirants, les déodorants, les vernis à ongles, les écrans solaires, les produits de protection des mains, les produits de régénération cutanée ou antivieillissement, les produits de régénération nocturne, les laits corporels, les produits pour le visage, les produits de soin de jour protecteurs, les hydratants, les shampooings, les gels capillaires, le mascara, et les sprays capillaires.

12. Composition selon la revendication 1, où la quantité d'émulsifiant constitue de 0,5 à 6% en poids de la composition.

13. Composition selon la revendication 1, où la composition comprend en outre au moins un élément choisi dans le groupe constitué par les triglycérides, les benzoates de C₁₂-C₁₅-alkyle, l'huile minérale et les huiles naturelles.

14. Composition selon la revendication 2, où l'huile de silicone comprend au moins une huile parmi la diméthicone et la cyclométhicone.

15. Composition selon la revendication 1, comprenant en outre au moins un élément choisi dans le groupe constitué par le glycérol, l'hexylène glycol, les gommes naturelles et les polysaccharides.

16. Méthode de préparation d'une composition de soin personnel, comprenant la combinaison des ingrédients selon la revendication 1.
